# FASCICULE DE BREVET EUROPEEN

(11) **EP 1 174 497 B1**
(45) Date de publication et mention de la délivrance du brevet: **08.09.2004**
(21) Numéro de dépôt: 00115568.8
(22) Date de dépôt: 19.07.2000
(51) Int. Cl.: C12M 3/06

(54) **Chambre de culture cellulaire et bioréacteur pour la culture extracorporelle de cellules animales**
Kulturraum und Bioreaktor zur ausserkörperlichen Tierzellenkultur
Culture chamber and bioreactor for the extracorporeal culture of animal cells

(43) Date de publication de la demande: 23.01.2002
(73) Titulaire: Technodop Ltd. (Société de Droit Irlandais), Dublin 2 (IE)
(72) Inventeur: Vetillard, Jérôme, 77185 Lognes (FR); Herodin, Francis, 38920 Crolles (FR); Caterini, Richard, 69530 Orliéans (FR)
(74) Mandataire: Gaucherand, Michel

(56) Documents cités:
- EP-A- 0 155 237
- EP-A- 0 363 262
- WO-A-90/01981
- WO-A-93/18133
- WO-A-98/30308
- US-A- 5 599 688
- US-A- 5 605 835

## Description

### Domaine de l'invention

La présente invention concerne une chambre de culture cellulaire et un bioréacteur la contenant pour la culture extracorporelle de cellules animales.

L'invention concerne plus particulièrement une chambre de culture cellulaire à au moins deux membranes planes filtrantes à seuil de coupure différent délimitée par une enveloppe à axe de symétrie et un bioréacteur qui permet la culture de cellules animales dans ladite chambre de culture tout en régulant et contrôlant l'environnement dans lequel les cellules sont cultivées.

La chambre de culture cellulaire et le bioréacteur de l'invention pour la culture extracorporelle de cellules animales permettent la culture de cellules animales dans des conditions stériles.

L'invention s'applique à la production de cellules animales comme par exemple les cellules hématopoïétiques, les cellules hépatiques, les cellules de la peau (appelées les kératinocytes), les cellules de pancréas, les cellules nerveuses organisées ou non en structure tissulaire dans un but thérapeutique.

### Etat de la technique

La transplantation d'organe, de tissus ou de cellules représentant une avancée technologique médicale considérable, un vif intérêt économique s'est par conséquent développé pour la réalisation de bioréacteurs destinés à la culture de cellules animales dans un but thérapeutique soit en greffant lesdites cellules, soit en les utilisant dans des dispositifs externes interfacés avec le patient comme moyens palliatifs à une déficience organique.

Un inconvénient majeur des bioréacteurs actuels destinés à la culture des cellules eucaryotes réside dans le transfert de masse, c'est-à-dire le transfert de masse des nutriments et de l'oxygène dissous jusqu'aux cellules eucaryotes, du fait que ces cellules sont fragiles et qu'elles sont détruites par le stress mécanique généré par l'agitation du milieu pour l'aérer.

Le brevet américain n° 6,048,721 décrit un bioréacteur pour la croissance et le maintien ex vivo de cellules mammifères. Dans ce bioréacteur, la chambre de culture discoïdale est délimitée par un lit planaire de cellules et une membrane perméable aux gaz et imperméable au liquide. Le milieu nourricier perfusé dans le compartiment inférieur du bioréacteur se diffuse radialement et l'air insufflé dans le compartiment supérieur oxygène le milieu. Selon le mode de fonctionnement de ce bioréacteur, le milieu chargé des déchets générés par la culture des cellules part à l'égout. La récupération des cellules après culture se fait selon un traitement enzymatique. Dans ce type de dispositif, l'épaisseur de la chambre de culture risque d'induire un gradient de pression partielle en oxygène qui est peu adapté à une bonne viabilité cellulaire.

L'inconvénient des bioréacteurs connus de l'art antérieur, par example celui décrit dans EP-A-0 155 237, et destinés à la culture des cellules eucaryotes réside dans le fait que ces bioréacteurs fonctionnent en perfusion continue de leur chambre de culture, le flux du milieu nourricier n'est pas récupéré et est directement déversé à l'égout augmentant de façon conséquente le prix de revient de la culture.

Or, pour la culture de cellules animales, telles que par exemple les cellules hématopoïétiques ou les cellules hépatiques, un flux nourricier riche en facteurs de croissance s'avère indispensable pour permettre la multiplication et la différenciation desdites cellules. Lesdits facteurs de croissance étant extrêmement coûteux, le fonctionnement d'un bioréacteur ne permettant pas de recycler ces facteurs représente un coût financier considérable, incompatible dans l'optique d'une exploitation clinique.

La présente invention a pour but de remédier à tous ces inconvénients.

### Objet de l'invention

Le but de la présente invention est de créer une chambre de culture et un bioréacteur pour la culture extracorporelle de cellules animales, permettant de conserver l'homéostasie du milieu environnant les cellules cultivées et ainsi leur permettre de proliférer dans les meilleures conditions possibles.

Pour parvenir à ce but, l'invention développe les objectifs ci-après exposés.

Un objet de l'invention est de maintenir une bonne viabilité cellulaire au sein de ladite chambre de culture et du bioréacteur, et ceci en fournissant d'une part aux cellules du milieu de culture un apport nutritionnel en quantité suffisante et en évacuant d'autre part les déchets et les inhibiteurs générés pour permettre une croissance de la population cellulaire.

Un autre objet de l'invention est de pouvoir recycler les facteurs de croissance du milieu tout en évacuant du milieu de culture suffisamment de déchets cellulaires et de réaliser ainsi l'optimisation économique de la culture des cellules.

Un autre objet de l'invention est de pouvoir effectuer un transfert de gène ciblé sur les cellules cultivées.

Un autre objet de l'invention est de maintenir les propriétés physico-chimiques du milieu de culture cellulaire en dépit de la perturbation induite par la croissance cellulaire.

Un autre objet de la présente invention est de garantir la stérilité, l'asepsie de la chambre de culture et du bioréacteur, et en particulier pendant toute la durée de la culture cellulaire.

Un autre objet de l'invention est de récupérer les cellules cultivées au sein de la chambre de culture et du bioréacteur de l'invention.

### Sommaire de l'invention

La présente invention est basée sur l'observation qu'une chambre de culture et un bioréacteur pour la culture extracorporelle de cellules animales pouvaient remédier aux différents inconvénients mentionnés ci-dessus s'ils permettaient à la fois de maintenir une bonne viabilité cellulaire des cellules cultivées tout en recyclant les facteurs de croissance du milieu, assurant ainsi un bon niveau de prolifération cellulaire.

Ainsi, l'invention a pour objet une chambre de culture pour la culture extracorporelle de cellules animales, délimitée par une enveloppe à axe de symétrie qui est formée d'une paroi latérale externe, de deux parois d'extrémités et d'entrées et de sorties de milieux liquides dynamiques, cette chambre étant caractérisée par le fait qu'elle comporte :
a) au moins deux membranes planes filtrantes à seuil de coupure différent, perpendiculaires à l'axe de symétrie ;
b) entre les membranes, un lit de macrosupports biocompatibles permettant l'adhésion des cellules en état de culture ;
c) deux parois d'extrémités pourvues de stries de distribution des milieux liquides dynamiques ;
d) trois couples d'entrées et de sorties des milieux liquides dynamiques, destinés à alimenter la chambre de culture des cellules et extraire sélectivement les cellules cultivées, les déchets résultant de leur culture et les nutriments en excès, dont deux des couples sont connectés aux parois d'extrémités pourvues de stries de distribution, le troisième étant connecté entre les deux membranes planes filtrantes.

L'invention a également pour objet un bioréacteur pour la culture extracorporelle de cellules animales comprenant une chambre de culture, délimitée par une enveloppe à axe de symétrie formée d'une paroi latérale externe, de deux parois d'extrémités et d'entrées et de sorties de milieux liquides dynamiques et comprenant des moyens de circulation desdits milieux dans ladite chambre, ce bioréacteur étant caractérisé en ce qu'il comporte :
a) une chambre de culture desdites cellules comportant au moins deux membranes planes filtrantes à seuil de coupure différent, perpendiculaires à l'axe de symétrie et qu'entre lesdites membranes à seuil de coupure différent se situe un lit de macrosupports biocompatibles favorisant l'adhésion des cellules en état de culture, ladite chambre étant délimitée par une enveloppe à axe de symétrie comprenant deux parois d'extrémités pourvues de stries de distribution des milieux liquides dynamiques et trois couples d'entrées et de sorties des milieux liquides dynamiques F1, F2, F3, destinés à alimenter la chambre de culture des cellules et à extraire sélectivement les cellules cultivées, les déchets résultant de leur culture et les nutriments en excès, dont deux des couples sont connectés aux parois d'extrémités pourvues de stries de distribution, le troisième étant connecté entre les deux membranes planes filtrantes ;
b) des moyens de circulation du premier milieu liquide dynamique F1 selon un circuit en boucle fermée et un vase d'expansion R1 contenant ledit milieu, ces moyens étant reliés à ladite chambre de culture ;
c) des moyens de circulation du deuxième milieu liquide dynamique F2 selon un circuit en boucle fermée ou en boucle ouverte suivant la fonction attribuée audit milieu et un réservoir R2 contenant ledit milieu, ces moyens étant reliés à ladite chambre de culture ;
d) des moyens de circulation du troisième milieu liquide dynamique F3 selon un circuit en boucle ouverte et un réservoir R3 contenant ledit milieu, ces moyens étant reliés à ladite chambre de culture ;
e) des moyens de contrôle et de régulation, et de conditionnement des milieux liquides dynamiques, reliés à un bloc de régulation-commande.

### Description des figures

- La figure 1 représente une coupe schématique d'une chambre de culture cellulaire délimitée par une enveloppe à axe de symétrie de forme hexagonale.
- La figure 2 représente schématiquement la circulation dynamique des milieux liquides F1 et F3 pour un gradient de pression p1>p3 en « phase descendante » au sein de la chambre de culture d'un bioréacteur de l'invention.
- La figure 3 représente schématiquement la circulation dynamique des milieux liquides F1 et F3 pour un gradient de pression p3>p1 en « phase ascendante » au sein de la chambre de culture d'un bioréacteur de l'invention.
- La figure 4 représente schématiquement un gradient de pression p1>p3 en « phase descendante » au sein de la chambre d'un bioréacteur.
- La figure 5 représente schématiquement un gradient de pression p3>p1 en « phase ascendante » au sein de la chambre de culture d'un bioréacteur selon l'invention.
- La figure 6 représente un schéma de montage d'un bioréacteur où le bloc de régulation-commande n'est pas représenté.

### Description détaillée de l'invention

L'invention concerne une chambre de culture cellulaire à axe de symétrie, renfermant à la fois les cellules et le milieu de culture, dans laquelle un lit de macrosupports biocompatibles est confiné entre deux membranes planes filtrantes à seuil de coupure différent, ladite chambre étant délimitée par une enveloppe à axe de symétrie formée d'une paroi latérale externe et de deux parois d'extrémités.

Selon l'invention, cette chambre de culture cellulaire comporte au moins deux membranes planes filtrantes à seuil de coupure différent et des macrosupports biocompatibles qui permettent l'adhésion des cellules en état de culture et qui sont situés entre deux desdites membranes à seuil de coupure différent.

Ainsi, une première membrane plane filtrante à seuil de coupure de l'ordre de 0,12 µm à 0,44 µm permet les échanges biochimiques au sein de la chambre de culture en autorisant le passage des molécules du milieu nourricier telles que les protéines et les macromolécules, mais réalisant un confinement cellulaire empêchant les cellules cultivées de quitter la zone d'homéostasie et empêchant également le passage des particules contaminantes en servant notamment de barrière aux bactéries pouvant contaminer ladite chambre.

Une autre membrane plane filtrante à seuil de coupure d'au plus 15 KiloDalton (KDa) autorise quant à elle le confinement moléculaire de toutes les molécules ayant une masse moléculaire supérieure à 15 KDa. La présence de cette membrane plane filtrante au sein de la chambre de culture permet de définir un espace de confinement des cellules de culture. Par conséquent, la membrane plane filtrante à seuil de coupure d'au plus 15 KDa confine les cellules en culture dans la chambre de culture, ainsi que les facteurs de croissance et les grosses protéines.

Selon une caractéristique de l'invention, au moins une des membranes a un seuil de coupure compris préférentiellement entre 0,18 µm et 0,26 µm et l'autre un seuil de coupure préférentiellement compris entre 10 et 12 KDa.

Selon l'invention, le nombre de membranes présentes dans la chambre de culture peut être supérieur à deux. Dans ce cas, les membranes supplémentaires ont des seuils de coupure adaptés à ceux des deux membranes précitées.

Selon l'invention, les membranes planes filtrantes à seuil de coupure différent au sein de la chambre de culture cellulaire sont disposées perpendiculairement par rapport à l'axe de symétrie de ladite chambre de culture.

Selon l'invention, les deux membranes planes filtrantes à seuil de coupure différent peuvent être des membranes minérales ou organiques. A titre d'exemple, on peut citer des membranes constituées de polymère organique biocompatible, de cellulose ou de polysulfone.

De plus, ces deux membranes planes filtrantes sont distantes l'une de l'autre d'au plus 400 µm, distance qui s'avère favorable à un bon développement des cellules puisqu'elles sont pratiquement toujours en contact avec les sources de nutriments et d'oxygène.

Entre les deux membranes filtrantes à seuil de coupure différent est confiné un lit de macrosupports biocompatibles dont les plus grosses particules qui peuvent avoir la forme de sphère ne doivent pas dépasser 400 µm en équivalent diamétral. Ce lit de macrosupports, constitué de particules de tailles diverses pouvant être éventuellement agglomérées en un bloc continu par frittage des éléments granulaires, ne doit pas avoir une épaisseur supérieure à 400 µm. Ledit lit de macrosupports joue d'une part un rôle de support des cellules en état de culture et d'autre part un rôle mécanique de maintien de l'espace de confinement cellulaire aménagé entre les deux membranes planes filtrantes à seuil de coupure différent. Suivant le type de cellules cultivées au sein de la chambre de culture de l'invention, le type de macrosupports biocompatibles sera choisi de façon appropriée et de taille adéquate.

Les macrosupports mis en oeuvre entre les deux membranes de la chambre de culture peuvent avoir une forme cylindrique ou sphérique ou encore polyédrique tel que des blocs massifs usinés dont l'épaisseur est d'au plus 400 µm.

Selon l'invention, les macrosupports peuvent être d'origine minérale (tel que, par exemple, le corail) ou métallique (tel que le titane et ses alliages par exemple) ou encore formés de polymères biocompatibles.

A titre illustratif, dans le cas d'une culture de cellules hématopoïétiques en vue d'une application pour une greffe de moelle osseuse d'une part et dans le cas d'une culture d'ostéoblastes dans un but de reconstruction osseuse d'autre part, les macrosupports peuvent être des microbilles de corail ayant un diamètre d'au plus 400 µm. En effet, des billes de corail de granulométrie souhaitée s'avèrent être des macrosupports appropriés pour les applications mentionnées ci-dessus du fait notamment de leur aptitude à être colonisées par les progéniteurs hématopoïétiques. Dans le cas des précurseurs osseux, les billes de corail seraient de plus métabolisées, ce qui favoriserait leur utilisation en chirurgie reconstructrice.

Alors que, par exemple, dans le cas d'une culture de cellules hépatiques en vue d'une application de détoxification sanguine ex-vivo d'un patient hépato-insuffisant par une biomasse extracorporelle d'hépatocytes, les macrosupports les plus adaptés peuvent être des billes de nylon® (polyamides) ayant un diamètre inférieur à 400 µm.

Ainsi, la présence de macrosupports entre lesdites membranes et le fait que la membrane plane filtrante à seuil de coupure d'au plus 15 KDa n'autorise pas le passage des cellules en culture de même que celui des facteurs de croissance, permettent le confinement des cellules en culture, qui adhèrent aux dits macrosupports biocompatibles, dans cet espace de confinement cellulaire situé entre les deux membranes à seuil de coupure différent.

L'alimentation en milieux liquides dynamiques de la chambre de culture est réalisée de façon homogène du fait de la répartition et de la distribution desdits milieux au sein de ladite chambre grâce à des entrées et des sorties de ces milieux judicieusement placées.

La chambre de culture cellulaire comme précédemment évoquée comporte une enveloppe à axe de symétrie formée d'une paroi latérale externe et de deux parois d'extrémités que l'on peut assimiler à deux fonds plats situés à chacune des extrémités de ladite paroi latérale.

Cette enveloppe à axe de symétrie peut être constituée par exemple d'un matériau polymère biocompatible ou d'acier inoxydable. A titre de matériaux polymères biocompatibles, on peut citer par exemple les polyoléfines, les polyamides ou les polyesters et autres.

Les deux parois d'extrémités de l'enveloppe à axe de symétrie sont pourvues de stries de distribution permettant d'alimenter la chambre de culture par deux des trois milieux liquides dynamiques. Ces stries de distribution constituent un réseau principal de stries dites principales. Ce réseau principal de stries situé sur la face de chacune des deux parois d'extrémités, en regard de l'espace de culture cellulaire, peut être divergent à partir de la tubulure d'entrée aménagée dans ladite paroi.

Selon l'invention, le réseau principal de stries dites principales, aussi appelé réseau de distribution permet une répartition homogène des deux milieux liquides dynamiques, qui sont acheminés vers la chambre de culture par l'intermédiaire de conduits biocompatibles dont la ou les extrémités sont enfichées au niveau des parois d'extrémités. Le nombre de stries de distribution situées sur la face en regard de l'espace de culture cellulaire de chacune des deux parois d'extrémités est défini de manière à obtenir une bonne dispersion du milieu liquide dynamique dans la chambre de culture et sera déterminé en fonction de la forme de l'enveloppe à axe de symétrie

Ledit réseau principal est complété par un réseau secondaire, formé de stries dites secondaires, moins profond et de direction sensiblement orthogonale par rapport au réseau principal de façon à favoriser la circulation entre les zones de distribution délimitées par le réseau principal. Ainsi, les stries secondaires du réseau secondaire sont sensiblement perpendiculaires aux stries du réseau principal. Ce réseau secondaire peut voir son espacement varier de telle sorte que les stries secondaires soient plus rapprochées et plus nombreuses du côté opposé à l'entrée du flux de milieu pour faciliter le drainage et l'évacuation dudit milieu.

Deux stries adjacentes du réseau principal constituent une zone de distribution et deux stries adjacentes du réseau principal recoupées par deux stries adjacentes du réseau secondaire constituent une alvéole de distribution.

Les réseaux principal et secondaire forment un fin réseau "quadrillé" de propagation des milieux liquides dynamiques. L'ensemble de ces deux réseaux peut par exemple former un réseau en forme de gaufre.

Le réseau principal étant situé sur la face en regard de l'espace de culture cellulaire, possède une certaine hauteur et une certaine largeur que l'homme du métier est tout à fait à même de pouvoir définir, sachant que le réseau de stries doit être solidaire de la membrane plane filtrante pour assurer la cohésion de l'ensemble. En effet, le volume ménagé par l'ensemble solidaire du réseau de stries et de la membrane forme le quadrillage de distribution du flux liquidien qui peut représenter environ 50% de la surface de la membrane.

Ainsi, le réseau principal peut être constitué de stries principales ayant une profondeur allant d'au plus 5 mm et une largeur d'au plus 2 mm, le pas compris entre deux stries adjacentes dudit réseau principal pouvant être d'au plus 2 mm. De même, le réseau secondaire peut être constitué de stries secondaires ayant une profondeur d'au plus 2 mm et une largeur d'au plus 2 mm, le pas compris entre deux stries adjacentes dudit réseau diminuant progressivement depuis le côté d'entrée du flux de milieu vers la sortie dudit flux de milieu. De ce fait, dans sa partie distale -c'est à dire du côté de la sortie du milieu- le pas du réseau secondaire sera d'au plus 2 mm.

A titre d'exemple, le réseau principal dans les parois d'extrémités peut en pratique avoir une profondeur de 1 mm et une largeur de 500 µm avec un pas de 500 µm et le réseau secondaire peut par exemple avoir une profondeur de 500 µm et une largeur de 500 µm avec un pas minimum de 500 µm.

Par conséquent, les parois d'extrémités peuvent être envisagées comme réalisant un fin motif d'alvéoles de type gaufre sur lequel les membranes à seuil de coupure différent viennent prendre appui ou sont collées par une colle biocompatible de type colle polymère.

L'enveloppe à axe de symétrie peut se présenter sous la forme de deux demi-enveloppes telles que par exemple deux demi-cylindres, constituées chacune d'une paroi latérale externe et d'une paroi d'extrémité, qui sont reliées l'une à l'autre de manière étanche au moyen de joints d'étanchéité.

La forme de l'enveloppe à axe de symétrie de la chambre de culture selon l'invention peut être choisie de façon appropriée pour faciliter par exemple un empilement de plusieurs chambres de culture sur un support. L'homme du métier sera encore une fois à même de choisir la forme de l'enveloppe à axe de symétrie de ladite chambre de culture selon l'usage qu'il en sera fait. A titre d'exemple, on peut envisager une enveloppe à axe de symétrie, constituée ou non de deux demi-enveloppes, de forme cylindrique, cubique ou polygonale.

Pour une enveloppe à axe de symétrie de forme hexagonale, par exemple, le nombre de stries principales de distribution de chacune des deux parois d'extrémités peut être d'au moins sept, ce nombre pouvant être beaucoup plus important.

Il convient de souligner qu'un empilement de chambres de culture à motifs élémentaires peut être réalisé à la condition que lesdits motifs aient la même forme pour obtenir un empilement stable de ces motifs sur un support adéquat.

Selon un mode d'exécution d'empilement de chambres de culture à motifs élémentaires, une forme par exemple hexagonale peut faciliter l'empilement successif de plusieurs chambres sur un socle approprié possédant par exemple six colonnes. Dans ce cas, les six colonnes du socle jouant le rôle de support pour l'empilement des chambres à motifs élémentaires peuvent également servir par exemple de conduits d'alimentation et d'évacuation des milieux liquides dynamiques.

Quant à l'alimentation en milieux liquides dynamiques de la chambre de culture selon l'invention, elle est réalisée par un système à trois milieux liquides dynamiques (à savoir un système à trois flux de milieux distincts).

Un premier milieu liquide dynamique noté F1, entrant et sortant par des tubulures biocompatibles enfichées au niveau d'une des parois d'extrémités de l'enveloppe à axe de symétrie (par exemple la paroi d'extrémité supérieure) alimente le milieu de culture en milieu nutritif (encore appelé milieu nourricier riche), ce milieu étant riche en facteurs de croissance. Ce premier milieu liquide dynamique est composé d'éléments nécessaires à la culture des cellules tels que par exemple de protéines, d'oligo-éléments, de glucose, d'eau et de facteurs de croissance, et alimente le milieu de culture en milieu nutritionnel frais.

Un deuxième milieu liquide dynamique noté F2 entrant et sortant au niveau de la paroi latérale externe de l'enveloppe à axe de symétrie de la chambre de culture peut présenter trois fonctions distinctes selon les usages qui en seront faits.

Selon une première fonction, ce deuxième milieu liquide dynamique, entrant par une tubulure biocompatible enfichée au niveau de la paroi latérale de la chambre de culture, sert à introduire dans ladite chambre les cellules destinées à être cultivées et à récupérer lesdites cellules cultivées au sein de la chambre après leur culture (cellules hématopoïétiques ou cellules hépatiques par exemple).

Selon une deuxième fonction, ce deuxième milieu liquide dynamique peut jouer le rôle de transfert de gènes. En effet, lors de l'introduction des cellules de culture en suspension dans la chambre de culture par l'intermédiaire de ce deuxième milieu liquide dynamique, des particules virales contenues dans ledit milieu liquide peuvent se fixer aux cellules en suspension au niveau de leurs membranes et permettre ainsi le transfert de gènes désiré. Ce deuxième flux de milieu peut permettre d'obtenir les cellules de culture souhaitées, génétiquement modifiées, que l'on désire cultiver. En effet, ce milieu transporte les vecteurs de transfert génique et permet leur mise en contact avec les cellules afin d'établir une fusion membranaire entre la cellule cible et le vecteur de transfert de gène. Ces vecteurs de transfert génique sont de toute nature. A titre d'illustration, on peut citer les virus comme par exemple les adénovirus, les rétrovirus, les liposomes, des complexes plasmidiques.

Un tel marquage des cellules (par l'injection de vecteurs de transfert de gène) réalisé en dehors du corps humain peut permettre de cibler avec précision le tissu à modifier génétiquement. De plus, on peut utiliser par exemple un virus synthétique à usage unique pour cibler le transfert de gène sur une population cellulaire d'intérêt thérapeutique. Ce virus caractérisé en ce que les gènes codant pour l'enveloppe et ceux qui véhiculent l'information génétique à proprement parler sont séparés, est incapable de se reproduire à l'intérieur des cellules après avoir transféré l'information génétique souhaitée. Ainsi les risques de genèse d'un virus recombinant à partir du virus synthétique utilisé et d'un virus sauvage qui pourrait être présent chez le patient sont limités.

Selon une troisième fonction, ce deuxième milieu liquide dynamique peut jouer le rôle de flux de rinçage des macromolécules inhibitrices présentes au niveau de l'espace de confinement cellulaire. En effet, les cellules mises en culture peuvent avoir subi un stress avant leur récolte ou lors de leur traitement pré-inoculaire ou encore pendant leur inoculation dans la chambre de culture. Ce stress peut induire la production (par lesdites cellules) de protéines qui vont inhiber leur capacité à se multiplier en les faisant passer dans un état de dormance, appelé état quiescent. Durant cet état, lesdites cellules ne sont plus en état physiologique de répondre à la stimulation réalisée par les facteurs de croissance, en se multipliant.

Dans le cas des cellules hématopoïétiques, on peut citer le stress « radio-induit » qui est provoqué par une exposition à des rayonnements ionisants (volontaires à usage radiothérapeutique ou accidentelles) et se traduit ultérieurement par un stress. Ce type de stress entraîne la production d'inhibiteurs tels que par exemple le « Transforming Growth Factor-béta » ou le « Tumor Necrosis Factor-alpha ». Ces molécules inhibitrices étant des cytokines au même titre que les facteurs de croissance apportés pour la culture de cellules hématopoïétiques, elles sont donc confinées par les membranes à seuil de coupure différent de la chambre de culture.

Pour effectuer une culture appropriée des cellules, il faut éliminer du milieu de culture et donc de la chambre de culture ces molécules inhibitrices. Le deuxième milieu liquide dynamique dans sa troisième fonction sert dès lors à rincer la zone de confinement cellulaire pour éliminer lesdites molécules inhibitrices.

Un troisième milieu liquide dynamique noté F3, entrant et sortant par des tubulures biocompatibles enfichées au niveau de la deuxième des parois d'extrémités de l'enveloppe à axe de symétrie (par exemple la paroi d'extrémité inférieure) alimente le milieu de culture en milieu nutritif de base (encore appelé milieu nutritif de base-régénérant-). Ce milieu nutritif de base est un milieu nourricier totalement dépourvu de facteurs de croissance. Un tel milieu de base est composé par conséquent de glucose, d'eau, d'oligo-éléments tels que par exemple des vitamines et des minéraux, de colorants pour évaluer le pH du milieu et de protéines de type albumine.

Selon l'invention, cette chambre de culture destinée à être alimentée par le système à trois milieux liquides dynamiques distincts (encore appelé triple flux) a pour caractéristique trois couples d'entrées et de sorties desdits milieux. Deux de ces couples sont connectés aux parois d'extrémités pour alimenter les stries de distribution. Le troisième couple est connecté à la paroi latérale externe à un niveau se situant entre les deux membranes planes filtrantes à seuil de coupure différent.

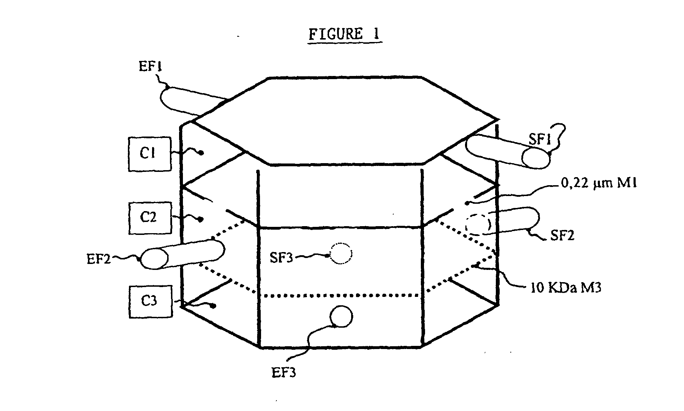

La disposition décalée de ces couples d'entrées et de sorties des milieux sur l'enveloppe délimitant la chambre de culture permet d'obtenir une homogénéité de distribution desdits milieux liquides au sein de ladite chambre (voir figure 1).

Selon les caractéristiques de l'invention, les tubulures d'entrées et de sorties du système triple flux sont par conséquent réparties de manière appropriée sur l'enveloppe à axe de symétrie de la chambre de culture.

Conformément à la figure 1, la tubulure biocompatible d'entrée du premier flux notée EF1 est enfichée au niveau de la paroi d'extrémité supérieure de l'enveloppe de la chambre de culture, alors que la tubulure de sortie notée SF1 du premier flux est également enfichée sur la même paroi d'extrémité mais à l'opposé de la tubulure d'entrée.

La tubulure biocompatible d'entrée du troisième flux notée EF3 est quant à elle enfichée au niveau de la paroi d'extrémité inférieure de ladite enveloppe de telle sorte que cette tubulure soit enfichée selon un angle d'environ 120 ° par rapport à la tubulure d'entrée du premier flux (EF1) nourricier riche en facteurs de croissance, pour permettre une bonne répartition desdits milieux au sein de la chambre de culture. Par ailleurs, la tubulure biocompatible de sortie de ce troisième flux notée SF3 est également enfichée sur la même paroi d'extrémité mais à l'opposé de la tubulure d'entrée dudit flux (EF3).

Conformément à la figure 1, la tubulure biocompatible d'entrée du deuxième milieu liquide dynamique notée EF2 est enfichée entre les deux membranes planes filtrantes notées M1 et M3, qui ont respectivement un seuil de coupure de 0,22 µm et de 10 KDa, au niveau de la paroi latérale externe de l'enveloppe selon un angle d'environ 60 ° par rapport à la tubulure d'entrée du premier flux (EF1). La tubulure biocompatible de sortie notée SF2 quant à elle est enfichée à l'opposé de la tubulure d'entrée dudit flux sur la paroi latérale.

En conséquence, les trois couples d'entrées et de sorties des milieux liquides dynamiques sont placés dans trois plans verticaux passant par l'axe de symétrie de l'enveloppe, ces plans étant décalés d'un angle d'environ 60° entre la première entrée et la deuxième entrée, et d'un angle d'environ 120° entre la première et la troisième entrée des milieux liquides dynamiques, les sorties desdits milieux liquides étant dans les mêmes dispositions angulaires.

La présente invention concerne également un bioréacteur comportant la chambre de culture cellulaire précédemment décrite. Cette chambre de culture au moyen de ces trois couples d'entrées et de sorties des milieux liquides dynamiques est reliée par des conduits de liaison à des réservoirs d'alimentation et/ou des réservoirs d'évacuation de ladite chambre. Le bioréacteur selon l'invention comporte en outre des moyens de régulation des conditions de culture et de contrôle de transfert de masse desdits milieux liquides dynamiques, reliés au bloc de régulation-commande dudit bioréacteur.

Ce bioréacteur comporte outre la chambre de culture « un bloc de régulation encore appelé bloc de commande » qui permet le contrôle et la régulation du pH, de la concentration en oxygène et de la température du milieu de culture. Le « bloc de régulation-commande » peut gérer en automatique le fonctionnement complet du bioréacteur. Cette régulation est de type P.I.D. Numérique (régulation numérique Proportionnelle, Intégrale et Dérivée) et les données renvoyées par les différentes sondes peuvent être enregistrées et analysées par informatique.

Ainsi, le bioréacteur de l'invention contenant la chambre de culture mentionnée précédemment, est organisé en modules unitaires fonctionnels interchangeables dimensionnés pour une certaine valeur de transfert de masse ou d'énergie (tels que, par exemple, des modules d'aération, des échangeurs thermiques), constituant un ensemble modulaire redimensionnable par juxtaposition d'unités fonctionnelles identiques en série et/ou en parallèle, et est doté d'un bloc de régulation-commande programmable.

Le bloc de régulation-commande reçoit l'ensemble des informations relatives aux milieux liquides dynamiques F1, F2, F3, par les moyens de contrôle et de régulation, ainsi que les informations relatives aux divers réservoirs, pompes, vannes et pressions régnant dans les zones C1, C2, C3 de la chambre de culture, les gère et émet les ordres de fonctionnement nécessaires.

De ce fait, il est possible de modifier à la demande les caractéristiques internes de la chambre de culture et de changer les paramètres et les programmes de régulation concourant à l'homéostasie du milieu afin de s'adapter au type cellulaire cultivé.

L'ensemble de régulation peut posséder également une cellule cristalline de mesure de spectre infrarouge, qui permet par déconvolution du spectre de réémission de mesurer la concentration de certains solutés dans le milieu de culture. Il est alors possible de suivre « en ligne » et « en temps réel » les concentrations de glucose et de lactate.

Ainsi, la température du milieu de culture peut être fixée à une valeur de consigne comprise entre 30 et 38 °C. Pendant la croissance cellulaire, le pH du milieu de culture peut être fixé à une valeur de consigne comprise entre 6,5 et 7,7. Le bloc de régulation-commande permet aussi de régler le débit d'air et le taux de CO₂ qui par sa dissolution donne l'amphotère HCO₃⁻ qui tamponne le milieu et concoure à la stabilité du pH et est l'effecteur de la régulation du pH.

Selon l'une des caractéristiques du bioréacteur de l'invention, les tubulures d'entrée et de sortie (de la chambre) du premier milieu liquide dynamique F1 sont reliées par des conduits de liaison à un réservoir R1 de milieu nourricier riche en facteurs de croissance selon un circuit en boucle fermée permettant le recyclage des facteurs de croissance nécessaires au développement des cellules en culture. Ce réservoir R1 peut jouer le rôle de vase d'expansion.

Selon l'une des caractéristiques de ce bioréacteur, ledit conduit de liaison entre la tubulure d'entrée du premier milieu nourricier riche F1 de la chambre et ledit réservoir noté R1 de ce milieu est équipé d'une pompe P1 permettant de contrôler et d'ajuster le volume et le débit du milieu F1 circulant en circuit fermé dans la chambre de culture.

La boucle fermée véhiculant le flux nourricier riche F1 dispose d'une mise à l'air qui se situe sur le vase d'expansion R1, cette mise à l'air est munie d'un filtre à seuil de coupure de 0,22 µm garantissant l'asepsie du milieu. De même, cette boucle est munie d'une électrovanne V1 commandée par le bloc de régulation-commande programmable permettant l'équilibrage de pression avec la pression atmosphérique à la demande. Le vase d'expansion R1 est également muni de capteurs de niveau haut et de niveau bas de milieu liquide servant à déclencher l'inversion de la circulation des fluides.

Selon une autre caractéristique du bioréacteur de l'invention, la tubulure d'entrée du troisième milieu nourricier de base (EF3) de la chambre de culture est reliée par un conduit de liaison à un réservoir noté R3 de ce milieu, et la tubulure de sortie (SF3) est reliée par un conduit à l'égout (compartiment de récupération des déchets).

Selon l'invention, le conduit de liaison relié à la tubulure d'entrée du troisième milieu liquide dynamique EF3 mentionnée ci-dessus est équipé d'une pompe P3 alors que le conduit relié à la tubulure de sortie dudit flux est équipé d'une vanne V3 éventuellement pilotée par le bloc de régulation-commande, l'ensemble permettant de régler et de contrôler le volume et le débit dudit troisième milieu F3 circulant en circuit ouvert dans la chambre de culture.

Selon l'invention, un dispositif aérateur du milieu nourricier riche F1 et un dispositif aérateur du milieu nourricier de base F3 sont prévus sur l'un et l'autre des deux circuits en boucles fermée et ouverte placés respectivement entre les entrées desdits milieux liquides dynamiques F1 et F3 dans la chambre de culture cellulaire et les réservoirs R1 et R3.

Selon l'invention, un échangeur thermique du milieu nourricier riche F1 et un échangeur thermique du milieu nourricier de base F3 sont prévus sur chacun des deux circuits en entrées desdits milieux liquides dynamiques F1 et F3 dans la chambre de culture cellulaire.

Ces dispositifs permettent de maintenir l'homéostasie de la chambre de culture en préconditionnant les flux de milieux qui y pénètrent afin de ne pas faire entrer dans la chambre de culture un élément de volume de milieu de culture pouvant induire une brutale perturbation de ses paramètres physico-chimiques.

Selon une autre caractéristique du bioréacteur de l'invention, la tubulure d'entrée du deuxième milieu liquide dynamique EF2 de la chambre de culture, située au niveau de la paroi latérale de l'enveloppe, est reliée par un conduit de liaison à un réservoir noté R2 contenant le deuxième milieu liquide dynamique choisi en fonction du rôle qui lui est attribué, à savoir soit alimenter la chambre de culture en cellules destinées à être cultivées et à décharger ladite chambre après culture, soit effectuer un transfert de gènes, soit avoir un rôle de flux de rinçage destiné à décharger la chambre de culture des molécules inhibitrices des cellules à cultiver.

Selon la fonction attribuée au deuxième milieu liquide dynamique de la chambre de culture, la tubulure de sortie SF2, située à l'opposé de la tubulure d'entrée EF2 sur ladite paroi, sera reliée :
- par un conduit de liaison à un réservoir de récupération de cellules après culture (à savoir une poche de récolte) ou à l'égout (compartiment d'élimination des molécules inhibitrices) selon un circuit en boucle ouverte ou
- par un conduit de liaison à un réservoir contenant le milieu pourvu de vecteurs appropriés de transfert de gène selon un circuit en boucle fermée.

Selon une autre caractéristique du bioréacteur de l'invention, un ou plusieurs modules fonctionnels échangeurs de milieux, encore appelés dialyseurs ou ultrafiltreurs, peuvent être ajoutés pour purifier le milieu nourricier F1 en sortie de la chambre de culture. Ce ou ces modules fonctionnels échangeurs de milieux sont situés à l'extérieur de la chambre de culture, montés en série sur le conduit de liaison de sortie du circuit en boucle fermée du premier milieu nourricier F1 riche en facteurs de croissance et parcourus à contre courant par le milieu nourricier F3 de base en boucle ouverte.

Dans le cas de l'utilisation de deux modules fonctionnels connectés en série de façon ad hoc sur la boucle fermée F1, on peut utiliser deux seuils de coupure différents de, par exemple, 10 KDa et de 30 KDa. On peut, en injectant le flux issu du module 10 KDa dans le module de 30 KDa, et en récupérant le perméat -c'est à dire la fraction liquide qui a traversé la membrane à seuil de coupure 30 KDa- pour le réinjecter dans la boucle F1, réaliser un fenêtrage des protéines du milieu entre 10 et 30 KDa qui correspond à la taille des facteurs de croissance.

De tels modules fonctionnels additionnels peuvent s'avérer utiles lorsque, par exemple, les facteurs de croissance sont produits par des cellules de support dans une première chambre de culture annexe, et que l'on désire conditionner ce milieu (c'est à dire récupérer les facteurs de croissance produits, sans récupérer les déchets cellulaires générés par ces cellules de supports) pour pouvoir le réutiliser dans la boucle F1 afin de stimuler les cellules dites d'intérêt thérapeutique présentes dans la chambre de culture principale.

Il convient de noter que le procédé utilisé pour cultiver des cellules hématopoïétiques et/ou sanguines, peut utiliser des cellules de support dites cellules stromales. Ces cellules stromales peuvent être modifiées génétiquement de façon à produire des cytokines humaines à des niveaux d'expression tels qu'ils peuvent subvenir pour partie aux besoins en cytokines de la culture.

Par ailleurs, il convient de souligner que l'enveloppe à axe de symétrie permettant d'obtenir une homogénéité de distribution des nutriments avec une dispersion optimale a l'avantage d'être stérile au même titre que tous les éléments du bioréacteur en contact avec les cellules et le milieu de culture. En effet, la stérilisation du bioréacteur est réalisée par autoclavage à 121 °C pendant 20 minutes de l'ensemble de l'appareil ainsi que des bouteilles de réservoirs. Les conduits de liaison, les réservoirs et autres éléments d'étanchéité sont réalisés en des matériaux biocompatibles pouvant supporter sans dommage une dizaine de cycles de stérilisation dans le cas d'une utilisation en laboratoire. Par contre, l'ensemble chambre de culture, conduits de liaison, réservoirs de milieux et autres constituera un kit de culture à usage unique dans le cas d'une utilisation en clinique humaine.

Par ailleurs, le reconditionnement du bioréacteur après une culture cellulaire, dans le cadre d'une utilisation de type laboratoire, se fait par une digestion protéique avec une solution d'acide chlorhydrique Molaire suivie d'un rinçage ultra pure, d'une re-stérilisation.

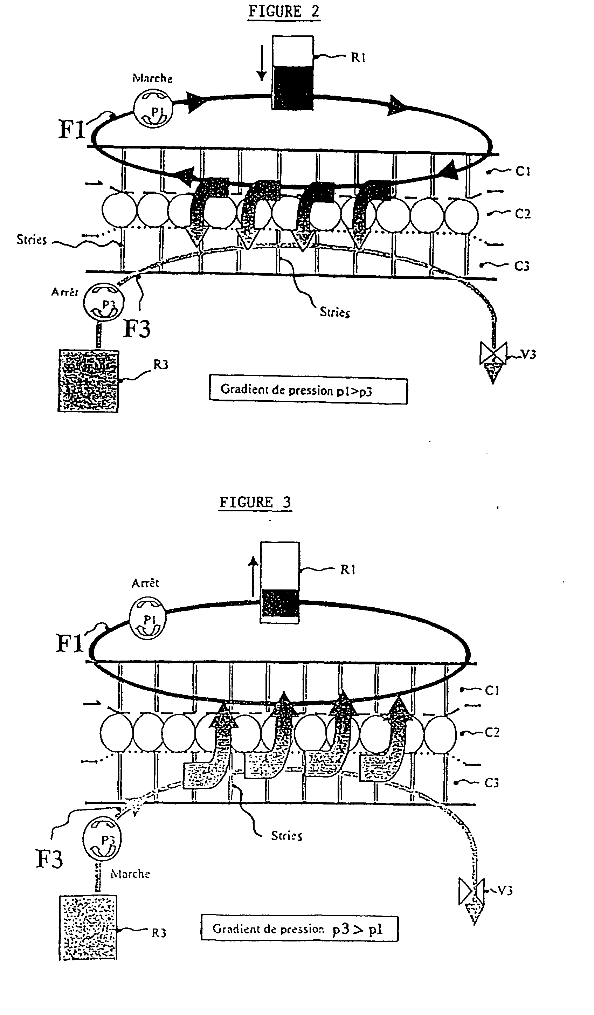

Selon le mode de fonctionnement du bioréacteur de l'invention et conformément à la figure 2, le milieu nourricier riche F1 est introduit au niveau de la paroi d'extrémité supérieure de l'enveloppe dans la zone notée C1 (compartiment C1) de la chambre de culture comprise entre ladite paroi et la membrane plane filtrante à seuil de coupure de l'ordre de 0,12 µm à 0,44 µm, par l'ouverture de la pompe P1 située sur le conduit de liaison reliant la chambre de culture au réservoir R1 contenant ledit milieu F1 alors que la pompe P3 située sur le conduit de liaison reliant la chambre de culture au réservoir R3 contenant le milieu nourricier de base F3 est à l'arrêt. De plus, la pression p3 régnant dans la zone notée C3 (compartiment C3) de la chambre de culture située entre la paroi d'extrémité inférieure de l'enveloppe et la membrane plane filtrante à seuil de coupure d'au plus 15 KDa, est réglée par la vanne de contre pression notée V3 située sur le conduit reliant la chambre de culture à l'égout de telle sorte que la pression p1 régnant dans la zone C1 de la chambre de culture est supérieure à la pression p3 et que la pression p2 régnant dans la zone notée C2 soit comprise entre p1 et p3 selon un gradient de pression.

Ainsi, lors de l'introduction du premier milieu liquide dynamique F1 dans la chambre de culture par l'ouverture de la pompe P1, les facteurs de croissance du milieu nutritif passent dans la zone notée C1 et au travers de ladite membrane à seuil de coupure de l'ordre de 0,12 µm à 0,44 µm de la chambre de culture, mais sont retenus par la membrane plane filtrante à seuil de coupure d'au plus 15 KDa, qui joue un rôle de barrière pour le passage des facteurs de croissance et des grosses protéines.

Par conséquent, les facteurs de croissance peuvent migrer de part et d'autre de la membrane plane filtrante à seuil de coupure de l'ordre de 0,12 µm à 0,44 µm assurant leur rôle de stimulation de la croissance et/ou de contrôle de la différenciation pour les cellules en état de culture confinées entre les deux membranes planes de la chambre de culture définissant la zone notée C2 (compartiment C2) de ladite chambre.

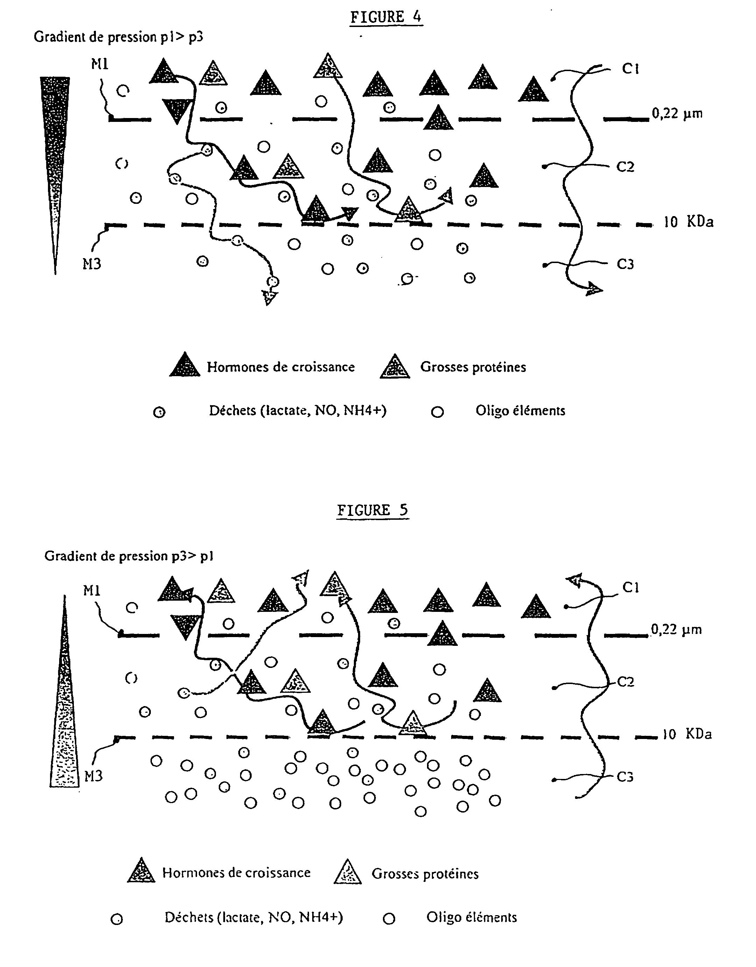

Conformément à la figure 4, la membrane plane filtrante M3 à seuil de coupure d'au plus 15 KDa confine les facteurs de croissance et les grosses protéines du milieu nutritif frais F1 dans les zones C1 et C2 de la chambre de culture. Par contre, les oligo-éléments dudit milieu nutritif F1 et les déchets de petites tailles (tels que, par exemple, NO, NH₄⁺, lactate et autres) générés par la culture des cellules confinées dans la zone C2 de ladite chambre, sont drainés vers la zone C3 où ils sont emportés vers l'égout. Le flux transmembranaire global est par conséquent orienté de la zone C1 vers la zone C3 de la chambre de culture (voir figure 2).

Dans ce mode de fonctionnement du bioréacteur selon l'invention, mode appelé « phase descendante », le milieu nutritif frais F1 circulant en boucle fermée perd de la volumétrie du fait du drainage de la solution aqueuse vers la zone C3 et notamment vers l'égout. Par conséquent, le volume de milieu nourricier F1 dans le réservoir R1 diminue. Les facteurs de croissance et les grosses protéines du milieu nutritif frais F1 étant confinés dans les zones C1 et C2, les déchets sont purgés des zones de la chambre de culture vers la zone C3 et sont drainés vers l'égout au moyen de la vanne V3 ouverte.

Dès que le volume du réservoir R1 ou vase d'expansion de la boucle fermée véhiculant le milieu nutritif frais F1 atteint un certain niveau bas, le bloc de régulation-commande du bioréacteur programmé selon une séquence particulière inverse la circulation de flux. De ce fait, la pompe P1 qui marchait passe à l'arrêt et la pompe P3 à l'arrêt se met en marche. Et, la vanne V3 ouverte est alors fermée.

Par conséquent, la pression p1 régnant dans la zone C1 de la chambre de culture devient inférieure à la pression p3, alors que la pression p2 régnant dans la zone notée C2 est comprise entre p1 et p3, selon un gradient de pression inversé par rapport au mode de fonctionnement précédent. Le flux transmembranaire global est alors orienté de la zone C3 vers la zone C2 de la chambre de culture (voir figure 3). Dans ce mode de fonctionnement du bioréacteur et conformément aux figures 3 et 5, mode appelé «phase ascendante», l'inversion de flux au sein de la chambre de culture permet au milieu nutritif frais F1 circulant dans la chambre de culture de se recharger en éléments nutritifs frais provenant le troisième milieu liquide dynamique F3 et de compenser les pertes occasionnées, entre autre l'eau, par la « phase descendante ». En effet, ce milieu de base(régénérant) F3 réalimente la chambre de culture cellulaire en glucose, en eau, en oligo-éléments.

Dès que le volume du réservoir R1 ou vase d'expansion du circuit en boucle fermée véhiculant le milieu nutritif frais F1 atteint un certain niveau haut, le système de contrôle du bioréacteur programmé selon une séquence particulière inverse à nouveau la circulation de flux.

Ainsi, les facteurs de croissance du fait de la circulation en boucle fermée du milieu nourricier riche F1, c'est-à-dire du premier milieu liquide dynamique dont ils font parties, sont confinés dans la boucle fermée et dans les compartiments C1 et C2, et leur concentration oscille entre une concentration C₀ et C₀+/-ΔC. Le milieu de culture riche est donc recyclé et l'utilisation des facteurs de croissance optimisée grâce à la chambre de culture et au bioréacteur de l'invention.

Ce système d'inversion de flux au sein de la chambre de culture permet de créer des flux transmembranaires présentant de faibles contraintes hydrodynamiques compatibles avec la fragilité des cellules cultivées. On obtient ainsi un système de flux lents « laminaires ». Cette inversion des flux peut également prévenir le colmatage des membranes filtrantes dont la perte de charge transmembranaire (indice mesurant la perméabilité de la membrane) pourra être contrôlée en temps réel par des manomètres électroniques placés sur chacun des flux de milieux liquides dynamiques.

Dans le mode de fonctionnement du bioréacteur de l'invention, le deuxième milieu liquide dynamique entrant et sortant au niveau de la paroi latérale externe, entre les deux membranes planes filtrantes de la chambre de culture, circule au sein de ladite chambre selon un circuit en boucle ouverte ou en boucle fermée suivant la fonction qu'il lui est attribuée, les pompes P1 et P3 étant à l'arrêt, et sa configuration d'entrée et sortie est fixée par la fonction qu'on veut lui attribuer.

Selon le mode de fonctionnement du bioréacteur, lorsque le deuxième milieu liquide dynamique F2 dans sa première fonction sert à inoculer les cellules à cultiver dans la chambre de culture et à les décharger après culture, il fonctionne en boucle ouverte au même titre que lorsqu'il sert dans sa troisième fonction à rincer la chambre pour éliminer les molécules inhibitrices. Par contre, lorsque le milieu liquide dynamique F2 a un rôle de transfert de gènes, il fonctionne en boucle fermée le temps de l'inoculation et de l'incubation.

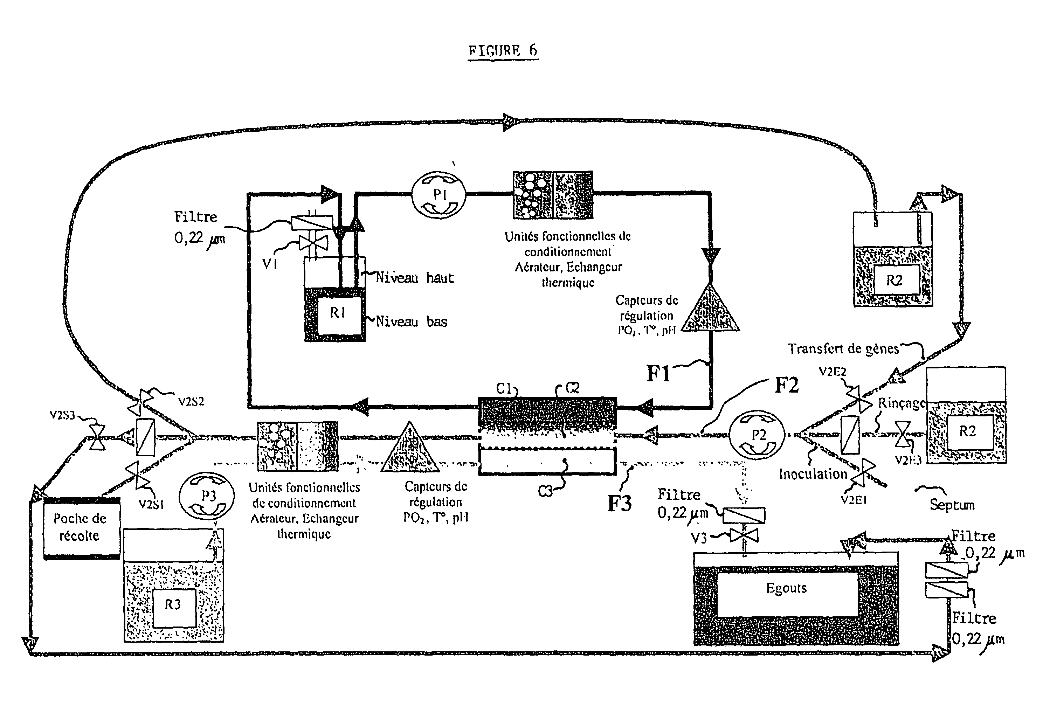

Dans sa première fonction et selon le mode de fonctionnement du bioréacteur conformément à la figure 6, le deuxième milieu liquide dynamique F2 contenant les cellules à cultiver est inoculé dans la zone C2 de la chambre de culture par une seringue au travers d'un septum biocompatible positionné sur l'une des trois branches de la tubulure d'entrée EF2 (de la paroi latérale externe) dont l'électrovanne V2E1 est ouverte, les électrovannes V2E2 et V2E3 étant fermées. Lors de l'inoculation dudit milieu, les trois électrovannes V2S1, V2S2 et V2S3 situées sur les trois branches de la tubulure de sortie SF2 aménagée à l'opposé de la tubulure d'entrée EF2 sont fermées.

Lors de la récupération des cellules après culture, les deux électrovannes V2E2 et V2E3 de la tubulure d'entrée EF2 sont fermées, l'électrovanne V2S1 située sur l'une des trois branches de la tubulure de sortie SF2 reliée à un conduit de liaison au réservoir de récupération des cellules cultivées, est ouverte alors que les électrovannes V2S2 et V2S3 des deux autres branches de la tubulure de sortie SF2 sont fermées. Le milieu de base régénérant est alors pompé par mise en route de la pompe P2 depuis le réservoir R2 et sert à purger le contenu du compartiment C2 (compris entre les deux membranes à seuil de coupure différent) dans la poche de collecte cellulaire. Un traitement enzymatique de type trypsine et/ou collagénase et/ou DNAse pourra être employé pour déstructurer la matrice extracellulaire produite par les cellules durant la culture pour faciliter leur ancrage. Dans le cadre, d'une culture de cellules hématopoïétiques sur un macrosupport de type corallien, une acidification légère du milieu à pH=6.0 ou pH=6.5 permettra de dissoudre le corail et de récupérer les cellules hématopoïétiques après rinçage.

Dans sa troisième fonction et selon le mode de fonctionnement du bioréacteur, lorsque le deuxième milieu liquide dynamique noté F2 contenant les éléments nécessaires au rinçage de l'espace de confinement cellulaire et fonctionnant en boucle ouverte selon le même principe de fermeture et d'ouverture des électrovannes que le fonctionnement décrit ci-dessus à l'exception que la tubulure d'entrée n'est pas munie d'un septum biocompatible mais est reliée par un conduit de liaison au réservoir R2 contenant ledit milieu F2 choisi.

Lors de l'introduction du milieu de rinçage F2 dans la zone C2 de la chambre de culture au niveau de l'une des trois branches de la tubulure d'entrée EF2 (de la paroi latérale externe) dont l'électrovanne V2E3 est ouverte, les électrovannes V2E1 et V2E2 étant fermées. Lors de l'introduction et de la récupération dudit milieu de rinçage, les deux électrovannes V2S1, V2S2 situées sur les deux branches de la tubulure de sortie SF2 sont fermées, l'électrovanne V2S3, située sur l'autre branche de la tubulure de sortie SF2 étant ouverte, le flux de rinçage circulant en continu en boucle ouverte. La sortie de l'électrovanne V2S3 peut être raccordée sur une tubulure menant à l'égout. L'égout étant constitué d'un réservoir de milieu stérilisé en même temps que l'ensemble des tubulures du bioréacteur, de ses réservoirs, de ses modules fonctionnels et de la chambre de culture, ce qui garantit la stérilité de l'écoulement. Deux filtres de 0,22 µm pourront être rajoutés sur ce tout à l'égout de façon à augmenter la sécurité et à garantir la stérilité, notamment lorsqu'il faudra changer "à chaud" le réservoir d'égout par la suite d'un trop plein.

Dans sa deuxième fonction et selon le mode de fonctionnement du bioréacteur conformément à la figure 6, le deuxième milieu liquide dynamique F2 contenant les vecteurs de transfert génique est introduit dans la zone C2 de la chambre de culture par l'ouverture de l'électrovanne V2E2 située sur l'une des trois branches de la tubulure d'entrée EF2, les électrovannes V2E1 et V2E3 étant fermées. Lors de l'introduction dudit milieu, les deux électrovannes V2S1, V2S3 situées sur deux branches de la tubulure de sortie SF2 sont fermées. Ce milieu F2 contenant les vecteurs de transfert génique circulant en circuit en boucle fermée le temps nécessaire à inoculation et à l'incubation. Lorsque la procédure de transfert de gènes est terminée, la boucle F2, repasse en mode de rinçage tel que décrit ci-dessus, afin de rincer les éventuels vecteurs résiduels. Puis, le rinçage étant fini, la culture se poursuit selon l'alternance des phases "ascendante" et "descendante" des flux F1 et F3.

Il convient de noter que l'un des épiphénomènes non désiré lors de l'inversion de flux de la phase descendante vers la phase montante est la possibilité que le milieu F1 soit légèrement contaminé par des déchets provenant des zones C2 et C3. Or il s'avère que le phénomène de dilution rend cette possible contamination négligeable. De plus, la partie proximale du conduit de liaison reliant le réservoir de milieu de base F3 et la chambre de culture est chargée de milieu F3 frais, c'est-à-dire provenant dudit réservoir, seule la partie distale du conduit reliant la chambre de culture à l'égout, c'est-à-dire près de la vanne V3, se charge en déchets.

La chambre de culture et le bioréacteur selon l'invention peuvent être utilisés pour la culture extracorporelle de cellules animales et, à ce titre, l'invention concerne le domaine médical. A titre illustratif, la chambre de culture et le bioréacteur selon l'invention peuvent s'appliquer à la production de cellules hématopoïétiques ou de cellules hépatiques.

La présente invention trouve ainsi son application pour la culture extracorporelle de cellules de la moelle osseuse. L'hématopoïèse est le processus physiologique qui permet de renouveler tous les éléments figurés du sang ( les cellules sanguines matures ayant une durée de vie limitée ) par la multiplication et la différenciation d'une cellule primitive originelle multipotente (appelée cellule souche) capable d'engendrer tous les types cellulaires, cellules sanguines encore appelées éléments figurés du sang. Ce processus est sous le contrôle de facteurs de croissance hématopoïétique, aussi appelées cytokines.

La greffe de cellules hématopoïétiques immatures, appelés progéniteurs hématopoïétiques, chez un patient soumis à une aplasie accidentelle ou consécutive à un traitement anticancéreux est un moyen de pouvoir relancer l'activité de la moelle osseuse lésée pour amorcer une reprise de l'hématopoïèse.

Ce processus implique que des progéniteurs -cellules à un stade précoce de développement- soient prélevés chez le patient par cytaphérèse ou directement par ponction de la moelle osseuse, et soient réinjectés après culture afin que ces cellules puissent reconstituer le système sanguin et immunitaire du patient (dans le cas d'une aplasie complète) ou compenser la période morbide de pancytopénie transitoire (phase de neutropénie, lymphopénie et thrombopénie) afin de permettre la reprise hématologique endogène de la victime aplasiée (dans le cas d'une irradiation accidentelle).

De même lors d'une insuffisance hépatique aiguë entraînant un risque vital à très brève échéance, il est nécessaire de pallier les fonctions hépatiques déficientes du patient par un traitement de détoxication sanguine extracorporelle nécessitant donc un grand nombre d'hépatocytes pour effectuer les fonctions métaboliques nécessaires. La chambre de culture ainsi que le bioréacteur décrits ci-dessus peuvent permettre la production de cellules hépatiques en nombre suffisant pour obtenir un traitement efficace et une rapidité de détoxication compatibles avec les contraintes hémodynamiques que pose la circulation extracorporelle du sang du patient.

A titre illustratif, un bioréacteur d'un volume utile qui peut varier de 50 à 100 ml permet de réaliser une culture extracorporelle de cellules animales sur une période d'une dizaine de jours dans le cadre de la production d'une biomasse cellulaire à usage de greffe et/ou comme moyen palliatif d'une déficience. Dans le cadre de la reconstitution d'un modèle tissulaire organisé et hiérarchisé, les durées de culture autorisées par le bioréacteur peuvent atteindre plusieurs mois.

Pour une greffe de cellules de type moelle osseuse (cellules hématopoïétiques), il est nécessaire que 10⁶ à 10⁷ de CFU (type de cellules immatures)/ Kg du poids du patient soient issues de la culture. Ce seuil clinique qui conditionne pour partie le succès de la greffe, permet d'estimer à 10¹⁰ le nombre de cellules issues de la culture hématopoïétique nécessaires à la greffe. La ou les chambres de culture ainsi que le bioréacteur tels que décrits peuvent être dimensionnés pour ce type d'application.

Les conditions de pH, de température et de pression partielle en oxygène pour une culture des cellules mentionnées ci-dessus seront de l'ordre de 7,4 pour un pH de croissance, de l'ordre de 37,5 °C pour la température et une pression partielle en oxygène (en % de saturation) égale à 15 %. Le milieu nourricier est un milieu synthétique constitué de protéines ultra purifiées ou synthétisées et d'oligo-éléments (tels que le fer, le sélénium, la transferrine, des vitamines et autres) et d'un colorant vital. Ce milieu nourricier est enrichi par des facteurs de croissance qui sont des cytokines dont la concentration peut varier de 10 à 100 ng/ml selon les besoins. De plus, les cytokines peuvent être stabilisées dans un état biologiquement actif avec la présence d'héparanes. Le milieu nourricier de base -régénérant- est commercialisé sous les dénominations commerciales suivantes, il peut être du MEM-alpha ou du RPMI1640 ou encore du IMDM.

## Revendications

1. Chambre de culture pour la culture extracorporelle de cellules animales, délimitée par une enveloppe à axe de symétrie qui est formée d'une paroi latérale externe, de deux parois d'extrémités et d'entrées et de sorties de milieux liquides dynamiques, **caractérisée en ce qu'**elle comporte :
a) au moins deux membranes planes filtrantes à seuil de coupure différent, perpendiculaires à l'axe de symétrie ;
b) entre les membranes, un lit de macrosupports biocompatibles favorisant l'adhésion des cellules en état de culture ;
c) deux parois d'extrémités pourvues de stries de distribution des milieux liquides dynamiques ;
d) trois couples d'entrées et de sorties des milieux liquides dynamiques, destinés à alimenter la chambre de culture des cellules et extraire sélectivement les cellules cultivées, les déchets résultant de leur culture et les nutriments en excès, dont deux des couples sont connectés aux parois d'extrémités pourvues de stries de distribution, le troisième étant connecté entre les deux membranes planes filtrantes.

2. Chambre de culture selon la revendication 1, **caractérisée en ce que** l'une des membranes planes filtrantes a un seuil de coupure compris entre 0,12 µm et 0,99 µm, et l'autre membrane plane filtrante a un seuil de coupure d'au plus 15 KiloDalton (KDa).

3. Chambre de culture selon l'une ou l'autre des revendications 1 et 2, **caractérisée en ce que** l'une des membranes planes filtrantes a un seuil de coupure compris entre 0,18 µm et 0,26 µm et l'autre membrane plane filtrante a un seuil de coupure compris entre 10 et 12 KDa.

4. Chambre de culture selon l'une au moins des revendications 1 à 3, **caractérisée en ce que** les deux membranes à seuil de coupure différent sont distantes l'une de l'autre d'au plus 400 µm.

5. Chambre de culture selon l'une au moins des revendications 1 à 4, **caractérisée en ce que** les macrosupports présents entre les membranes ont une forme cylindrique, ou polyédrique ou sphérique.

6. Chambre de culture selon l'une au moins des revendications 1 à 5, **caractérisée en ce que** la matière constitutive des macrosupports est choisie dans le groupe des matières minérales, des matières métalliques et des matières polymères.

7. Chambre de culture selon la revendication 6, **caractérisée en ce que** la matière constitutive des macrosupports est choisie dans le groupe constitué par le corail, le titane et ses alliages, les polyamides.

8. Chambre de culture selon l'une au moins des revendications 1 à 7, **caractérisée en ce que** le lit formé de macrosupports placé entre deux membranes à seuil de coupure différent, a une épaisseur d'au plus 400 µm.

9. Chambre de culture selon l'une au moins des revendications 1 à 8, **caractérisée en ce que** les stries de distribution des parois d'extrémités sont organisées en un réseau principal, ces stries étant préférentiellement divergentes dans le sens de propagation des milieux liquides dynamiques.

10. Chambre de culture selon l'une au moins des revendications 1 à 9, **caractérisée en ce que** les stries de distribution des parois d'extrémités sont complétées par un réseau secondaire formé de stries secondaires qui sont sensiblement perpendiculaires aux stries du réseau principal.

11. Chambre de culture selon l'une ou l'autre des revendications 9 et 10, **caractérisée en ce que** les réseaux principal et secondaire constituent un réseau quadrillé de propagation des milieux liquides dynamiques.

12. Chambre de culture selon l'une au moins des revendications 1 à 11, **caractérisée en ce que** les stries du réseau principal et/ou du réseau secondaire des parois d'extrémités sont au contact des membranes à seuil de coupure différent.

13. Chambre de culture selon l'une au moins des revendications 9 à 12, **caractérisée en ce que** les stries du réseau principal ont une profondeur d'au plus 5 mm, une largeur d'au plus 2 mm, et le pas compris entre deux stries adjacentes est d'au plus 2 mm.

14. Chambre de culture selon l'une au moins des revendications 9 à 12, **caractérisée en ce que** les stries du réseau secondaire ont une profondeur d'au plus 2 mm, une largeur d'au plus 2 mm.

15. Chambre de culture selon l'une au moins des revendications 1 à 14, **caractérisée en ce que** l'un des deux couples d'entrées et de sorties F1 des milieux liquides dynamiques est connecté au niveau d'une paroi d'extrémité et alimente le milieu de culture des cellules, par les stries de distribution, en un milieu nutritif comportant des facteurs de croissance par la traversée de la membrane plane filtrante à seuil de coupure compris entre 0,12 et 0,44 µm.

16. Chambre de culture selon la revendication 15, **caractérisée en ce que** le couple d'entrée et de sortie du milieu liquide dynamique F1 fonctionne en circuit fermé selon une boucle.

17. Chambre de culture selon l'une au moins des revendications 1 à 16, **caractérisée en ce que** le couple d'entrée et de sortie du milieu liquide dynamique F2, connecté entre les deux membranes filtrantes, assume les fonctions d'introduction des cellules à cultiver et de récupération desdites cellules cultivées, de transfert de gènes et de récupération des cellules génétiquement modifiées, et d'introduction d'un liquide de rinçage et d'élimination des molécules inhibitrices du développement des cellules en culture.

18. Chambre de culture selon l'une au moins des revendications 1 à 17, **caractérisée en ce que** l'autre des deux couples d'entrées et de sorties du milieu liquide dynamique F3 est connecté en circuit ouvert au niveau de l'autre paroi d'extrémité et alimente, par les stries de distribution, le milieu de culture des cellules en un milieu nutritif dépourvu de facteurs de croissance, par la traversée de la membrane plane filtrante à seuil de coupure d'au plus 15 KDa.

19. Chambre de culture selon l'une au moins des revendications 1 à 18, **caractérisée en ce que** les trois couples d'entrées et de sorties des milieux liquides dynamiques sont placés dans trois plans verticaux passant par l'axe de symétrie de l'enveloppe, ces plans étant décalés d'un angle d'environ 60° entre la première entrée et la deuxième entrée, et d'un angle d'environ 120° entre la première et la troisième entrée des milieux liquides dynamiques, les sorties desdits milieux liquides étant dans les mêmes dispositions angulaires.

20. Bioréacteur pour la culture extracorporelle de cellules animales comprenant une chambre de culture délimitée par une enveloppe à axe de symétrie formée d'une paroi latérale externe, de deux parois d'extrémités et d'entrées et de sorties de milieux liquides dynamiques et comprenant des moyens de circulation desdits milieux dans ladite chambre, **caractérisé en ce qu'**il comporte :
a) une chambre de culture desdites cellules comportant au moins deux membranes planes filtrantes à seuil de coupure différent, perpendiculaires à l'axe de symétrie et qu'entre lesdites membranes à seuil de coupure différent se situe un lit de macrosupports biocompatibles favorisant l'adhésion des cellules en état de culture, ladite chambre étant délimitée par une enveloppe à axe de symétrie comprenant deux parois d'extrémités pourvues de stries de distribution des milieux liquides dynamiques et trois couples d'entrées et de sorties des milieux liquides dynamiques F1, F2, F3, destinés à alimenter la chambre de culture des cellules et à extraire sélectivement les cellules cultivées, les déchets résultant de leur culture et les nutriments en excès, dont deux des couples sont connectés aux parois d'extrémités pourvues de stries de distribution, le troisième étant connecté entre les deux membranes planes filtrantes ;
b) des moyens de circulation du premier milieu liquide dynamique F1 selon un circuit en boucle fermée et un vase d'expansion R1 contenant ledit milieu, ces moyens étant reliés à ladite chambre de culture ;
c) des moyens de circulation du deuxième milieu liquide dynamique F2 selon un circuit en boucle fermée ou en boucle ouverte suivant la fonction attribuée audit milieu et un réservoir R2 contenant ledit milieu, ces moyens étant reliés à ladite chambre de culture ;
d) des moyens de circulation du troisième milieu liquide dynamique F3 selon un circuit en boucle ouverte et un réservoir R3 contenant ledit milieu, ces moyens étant reliés à ladite chambre de culture ;
e) des moyens de contrôle et de régulation, et de conditionnement des milieux liquides dynamiques, reliés à un bloc de régulation-commande.

21. Bioréacteur selon la revendications 20, **caractérisé en ce que** les moyens de circulation desdits milieux liquides F1, F2 et F3 sont équipés respectivement d'une pompe P1, P2 et P3, reliées au bloc de régulation-commande.

22. Bioréacteur selon les revendications 20 et 21, **caractérisé en ce que** les moyens de circulation du troisième milieu liquide dynamique F3 sont équipés d'une vanne V3 reliée au bloc de régulation-commande.

23. Bioréacteur selon la revendication 20, **caractérisé en ce que** le vase d'expansion R1 contenant le milieu liquide dynamique F1 riche en facteurs de croissance est muni d'un filtre ayant un seuil de coupure de 0,22 µm et d'une électrovanne V1 commandée par le bloc de régulation-commande.

24. Bioréacteur selon l'une au moins des revendications 20 à 23, **caractérisé en ce que** le vase d'expansion R1 est muni de capteurs de niveau haut et de niveau bas de milieu liquide, qui servent à déclencher l'inversion de la circulation des fluides au sein de ladite chambre de culture.

25. Bioréacteur selon la revendication 20, **caractérisé en ce que** les moyens de conditionnement des milieux liquides dynamiques comportent des moyens de réglage en oxygène, en température et en pH desdits milieux.

26. Bioréacteur selon la revendication 25, **caractérisé en ce que** les moyens de conditionnement desdits milieux sont des modules unitaires fonctionnels, dimensionnés pour une certaine valeur de transfert de masse ou d'énergie thermique.

27. Bioréacteur selon la revendication 26, **caractérisé en ce que** les modules fonctionnels sont des aérateurs, des échangeurs thermiques, des régulateurs de pH, ou encore des modules de dialyse, d'ultrafiltration.

28. Bioréacteur selon les revendications 20 à 27, **caractérisé en ce qu'**il comporte une chambre de culture selon l'une au moins des revendications 1 à 19.

29. Bioréacteur selon l'une au moins des revendications 20 à 28, **caractérisé en ce que** une pression p1 règne dans la zone C1 de la chambre de culture, située entre la paroi d'extrémité supérieure et la membrane plane filtrante à seuil de coupure compris entre 0,12 µm à 0,44 µm, de ladite chambre.

30. Bioréacteur selon l'une au moins des revendications 20 à 29, **caractérisé en ce que** une pression p2 règne dans la zone C2 de la chambre de culture comprise entre les deux membranes planes filtrantes à seuil de coupure différent de ladite chambre.

31. Bioréacteur selon l'une au moins des revendications 20 à 30, **caractérisé en ce que** une pression p3 règne dans la zone C3 de la chambre de culture, située entre la membrane plane filtrante à seuil de coupure d'au plus 15 KDa et la paroi d'extrémité inférieure de l'enveloppe de ladite chambre.

32. Bioréacteur selon l'une au moins des revendications 20 à 31, **caractérisé en ce que** le milieu nourricier riche en facteurs de croissance F1 est drainé de la zone C1 de la chambre de culture vers la zone C3 de ladite chambre de culture lorsque la pression p1 est supérieure à la pression p3 et que la pression p2 est comprise entre p1 et p3 dans la chambre de culture.

33. Bioréacteur selon l'une au moins des revendications 20 à 31, **caractérisé en ce que** le milieu nourricier de base dépourvu en facteurs de croissance F3 est drainé de la zone C3 de la chambre de culture vers la zone C1 de ladite chambre de culture lorsque la pression p3 est supérieure à la pression p1 et que la pression p2 est comprise entre p1 et p3 dans la chambre de culture.

34. Bioréacteur selon l'une au moins des revendications 20 à 32, **caractérisé en ce que** l'ouverture de la pompe P1 permet l'alimentation de la chambre de culture en milieu nourricier F1 riche en facteurs de croissance, la pompe P3 étant à l'arrêt, et que la vanne V3 de contre pression est réglée de telle sorte que la pression p1 régnant dans la zone C1 de ladite chambre de culture est supérieure à la pression p3 régnant dans la zone C3 de ladite chambre, sachant que la pression p2 régnant dans la zone C2 est comprise entre les pressions p1 et p3.

35. Bioréacteur selon la revendication 34, **caractérisé en ce que** le bloc de régulation-commande programmé selon une séquence particulière inverse le sens de circulation des flux en arrêtant la pompe P1 et en mettant en marche la pompe P3 dès que le volume contenu dans le vase d'expansion R1 atteint un niveau bas.

36. Bioréacteur selon la revendication 35, **caractérisé en ce que** la vanne V3 est fermée.

37. Bioréacteur selon les revendications 35 et 36 **caractérisé en ce que** le milieu nourricier de base F3 dépourvu de facteurs de croissance permet la réalimentation la chambre de culture où la pression p3 dans la zone C3 de ladite chambre est supérieure à la pression p1 régnant dans la zone C1, sachant que la pression p2 régnant dans la zone C2 est comprise entre les pressions p1 et p3.

38. Bioréacteur selon l'une au moins des revendications 20 à 37, **caractérisé en ce que** les pompes P1 et P3 sont à l'arrêt lorsque la configuration d'entrée et de sortie de circulation du deuxième milieu liquide dynamique F2 est fixée en un circuit fermé ou ouvert selon la fonction qu'il lui est attribuée.

39. Bioréacteur selon la revendication 38, **caractérisé en ce que** le milieu liquide dynamique F2 sert à l'introduction dans la chambre de culture des cellules à cultiver et à la récupération des cellules après culture.

40. Bioréacteur selon la revendication 38, **caractérisé en ce que** le milieu liquide dynamique F2 sert à l'introduction de vecteur de transfert de gènes dans la chambre de culture contenant les cellules en culture et à la récupération des cellules cultivées, génétiquement modifiées.

41. Bioréacteur selon la revendication 38, **caractérisé en ce que** le milieu liquide dynamique F2 sert à l'introduction d'un liquide de rinçage des molécules inhibitrices du développement des cellules en culture et à l'élimination desdites molécules.

42. Bioréacteur selon la revendication 39, **caractérisé en ce que** pour l'inoculation dudit milieu F2 dans la zone C2 de la chambre de culture cellulaire au travers d'un septum biocompatible à l'aide d'une seringue, l'électrovanne V2E1 est ouverte, alors que les électrovannes V2E2, V2E3, V2S1, V2S2 et V2S3 sont fermées.

43. Bioréacteur selon la revendication 39, **caractérisé en ce que** pour la récupération des cellules après culture dans la chambre de culture, l'électrovanne V2S1 est ouverte, les électrovannes V2E2, V2E3, V2S2, et V2S3 sont fermées et la pompe P2 est mise en route pour purger le contenu de la zone C2 dans la poche de collecte cellulaire.

44. Bioréacteur selon la revendication 41, **caractérisé en ce que** pour l'élimination des molécules inhibitrices lorsque le deuxième milieu F2 contient les éléments nécessaires au rinçage de l'espace de confinement cellulaire, lors de l'introduction dudit milieu l'électrovanne V2E3 est ouverte et les électrovannes V2E1, V2E2 sont fermées.

45. Bioréacteur selon la revendication 41, **caractérisé en ce que** ce deuxième milieu F2 de rinçage circule en continu en boucle ouverte et que lors de l'introduction et de la récupération dudit milieu de rinçage les électrovannes V2E1, V2E2, V2S1, V2S2 sont fermées, les électrovannes V2E3, V2S3 sont ouvertes.

46. Bioréacteur selon la revendication 40, **caractérisé en ce que**, pour l'inoculation des vecteurs de transfert génique contenu dans ledit milieu F2 circulant en circuit fermé dans la zone C2 de la chambre de culture cellulaire, l'électrovanne V2E2 est ouverte, alors que les électrovannes V2E1, V2E3, V2S1, V2S3 sont fermées.

47. Bioréacteur selon l'une au moins des revendications 20 à 46, **caractérisé en ce que** le bloc de régulation-commande reçoit l'ensemble des informations relatives aux milieux liquides dynamiques F1, F2, F3, par les moyens de contrôle et de régulation, et les informations relatives aux divers réservoirs, pompes, vannes et pressions régnant dans les zones C1, C2, C3 de la chambre de culture, les gère et émet les ordres de fonctionnement nécessaires.

## Patentansprüche

1. Kulturraum zur außerkörperlichen Tierzellenkultur, abgegrenzt durch eine Umhüllung, die eine Symmetrieachse aufweist, und gebildet durch eine äußere Seitenwand, zwei Endwände und Eingänge und Ausgänge für dynamische flüssige Medien,
**dadurch gekennzeichnet, dass** sie umfasst:
a) wenigstens zwei plane Filtermembrane mit unterschiedlicher Durchlassschwelle, senkrecht zu der Symmetrieachse;
b) zwischen den Membranen, ein biokompatibeles Makroträgerbett, das die Haftung der Zellen im Kulturzustand begünstigt;
c) zwei Endwände mit Verteilungsrillen der dynamischen flüssigen Medien;
d) drei Eingangs- und Ausgangspaare der dynamischen flüssigen Medien, bestimmt zur Versorgung des Kulturraums der Zellen und zur selektiven Entnahme der kultivierten Zellen, der aus ihrer Kultivierung resultierenden Abfälle und der überschüssigen Nährstoffe, wobei zwei der genannten Paare an die Verteilungsrillen aufweisenden Endwänden angeschlossen sind und das dritte zwischen den beiden planen Filtermembranen angeschlossen ist.

2. Kulturraum nach Anspruch 1, **dadurch gekennzeichnet, dass** eine der planen Filtermembrane eine Durchlassschwelle zwischen 0,12 µm und 0,44 µm hat, und die andere plane Filtermembran eine Durchlassschwelle von höchstens 15 Kilodalton (KDa) hat.

3. Kulturraum nach einem der Ansprüche 1 und 2, **dadurch gekennzeichnet, dass** eine der planen Filtermembrane eine Durchlassschwelle zwischen 0,18 µm und 0,26 µm hat, und die andere plane Filtermembran eine zwischen 10 und 12 KDa enthaltene Durchlassschwelle hat.

4. Kulturraum nach wenigstens einem der Ansprüche 1 bis 3, **dadurch gekennzeichnet, dass** die beiden Membrane mit unterschiedlicher Durchlassschwelle einen Abstand von höchstens 400 µm haben.

5. Kulturraum nach wenigstens einem der Ansprüche 1 bis 4, **dadurch gekennzeichnet, dass** die zwischen den Membranen vorhandenen Makroträger eine zylindrische oder polyedrische oder sphärische Form aufweisen.

6. Kulturraum nach wenigstens einem der Ansprüche 1 bis 5, **dadurch gekennzeichnet, dass** das die Makroträger bildende Material ausgewählt wird aus der Gruppe der mineralischen Materialien, der metallischen Materialien und der polymeren Materialien.

7. Kulturraum nach Anspruch 6, **dadurch gekennzeichnet, dass** das die Makroträger bildende Material ausgewählt wird aus der Gruppe, die gebildet wird durch Korallen, Titan und seine Legierungen, Polyamide.

8. Kulturraum nach wenigstens einem der Ansprüche 1 bis 7, **dadurch gekennzeichnet, dass** das durch Makroträger gebildete und zwischen zwei Membranen mit unterschiedlicher Durchlassschwelle befindliche Bett eine Dicke von höchstens 400 µm hat.

9. Kulturraum nach wenigstens einem der Ansprüche 1 bis 8, **dadurch gekennzeichnet, dass** die Verteilungsrillen der Endwände ein Hauptnetz bilden, wobei diese Rillen vorzugsweise in der Ausbreitungsrichtung der dynamischen flüssigen Medien divergieren.

10. Kulturraum nach wenigstens einem der Ansprüche 1 bis 9, **dadurch gekennzeichnet, dass** die Verteilungsrillen der Endwände durch ein Sekundämetz vervollständigt werden, das durch Sekundärrillen gebildet wird, die im Wesentlichen senkrecht sind zu den Rillen des Hauptnetzes.

11. Kulturraum nach dem einem oder dem anderen der Ansprüche 9 und 10, **dadurch gekennzeichnet, dass** das Hauptnetz und des Sekundämetz ein Gittemetz zur Ausbreitung der dynamischen flüssigen Medien bilden.

12. Kulturraum nach wenigstens einem der Ansprüche 1 bis 11, **dadurch gekennzeichnet, dass** die Rillen des Hauptnetzes und/oder des Sekundämetzes der Endwände in Kontakt sind mit den Membranen mit unterschiedlicher Durchlassschwelle.

13. Kulturraum nach wenigstens einem der Ansprüche 1 bis 12, **dadurch gekennzeichnet, dass** die Rillen des Hauptnetzes eine Tiefe von wenigstens 5 mm und eine Breite von höchstens 2 mm haben, und dass der zwischen zwei benachbarten Rillen enthaltene Abstand höchstens 2 mm beträgt.

14. Kulturraum nach wenigstens einem der Ansprüche 1 bis 12, **dadurch gekennzeichnet, dass** die Rillen des Sekundämetzes eine Tiefe von höchstens 2 mm und eine Breite von höchstens 2 mm haben.

15. Kulturraum nach wenigstens einem der Ansprüche 1 bis 14, **dadurch gekennzeichnet, dass** eines der beiden Eingangs- und Ausgangspaare F1 der dynamischen flüssigen Medien in gleicher Höhe einer Endwand angeschlossen ist und den Nährboden der Zellen durch die Verteilungsrillen mit einem Wachstumsfaktoren umfassenden Nährmedium versorgt, durch die plane Filtermembran mit einer zwischen 0,12 und 0,44 enthaltenen Durchlassschwelle hindurch.

16. Kulturraum nach Anspruch 15, **dadurch gekennzeichnet, dass** das Eingangs- und Ausgangspaar des dynamischen flüssigen Mediums F1 als geschlossener Kreislauf gemäß einer Schleife funktioniert.

17. Kulturraum nach wenigstens einem der Ansprüche 1 bis 16, **dadurch gekennzeichnet, dass** das Eingangs- und Ausgangspaar des dynamischen flüssigen Mediums F2, angeschlossen zwischen den beiden Filtermembranen, die Funktionen der Einführung der zu kultivierenden Zellen und der Rückgewinnung der kultivierten Zellen, des Transfers der Gene und der Rückgewinnung der genetisch modifizierten Zellen, und der Einführung einer Flüssigkeit zum Spülen und Eliminieren der die Entwicklung der Zellkultur hemmenden Moleküle.

18. Kulturraum nach wenigstens einem der Ansprüche 1 bis 17, **dadurch gekennzeichnet, dass** das andere der beiden Eingangs- und Ausgangspaare des dynamischen flüssigen Mediums F3 als offener Kreislauf in Höhe der anderen Endwand angeschlossen ist und durch die Verteilungsrillen den Nährboden der Zellen mit einem Nährmedium ohne Wachstumsfaktoren versorgt, durch die plane Filtermembran mit der Durchlassschwelle von höchstens 15 kDa hindurch.

19. Kulturraum nach wenigstens einem der Ansprüche 1 bis 18, **dadurch gekennzeichnet, dass** die drei Eingangs- und Ausgangspaare der dynamischen flüssigen Medien sich in drei vertikalen Ebenen befinden, durchquert von der Symmetrieachse der Umhüllung, wobei diese Ebenen zwischen dem ersten Eingang und dem zweiten Eingang der dynamischen flüssigen Medien um einen Winkel von ungefähr 60° versetzt sind und zwischen dem ersten und dem dritten Eingang um einen Winkel von ungefähr 120°, wobei für die Ausgänge in Bezug auf die Winkel dieselbe Anordnung gilt.

20. Bioreaktor zur außerkörperlichen Tierzellenkultur, mit einem Kulturraum, abgegrenzt durch eine Umhüllung, die eine Symmetrieachse aufweist, und gebildet durch eine äußere Seitenwand, zwei Endwände und Eingänge und Ausgänge für dynamische flüssige Medien, und mit Zirkulationseinrichtungen der genannten Medien in dem genannten Kulturraum,
**dadurch gekennzeichnet, dass** er umfasst:
a) einen Kulturraum der genannten Zellen mit wenigstens zwei planen Filtermembranen mit unterschiedlicher Durchlassschwelle, senkrecht zu der Symmetrieachse, und zwischen den Membranen mit unterschiedlicher Durchlassschwelle ein biokompatibles Makroträgerbett, das die Haftung der Zellen im Kulturzustand begünstigt, wobei der genannte Kulturraum durch eine Umhüllung mit Symmetrieachse abgegrenzt wird, die zwei Endwände mit Verteilungsrillen der dynamischen flüssigen Medien umfasst und drei Eingangs- und Ausgangspaare der dynamischen flüssigen Medien F1 ,F2 und F3, bestimmt zur Versorgung des Kulturraums der Zellen und zur selektiven Entnahme der kultivierten Zellen, der aus ihrer Kultur resultierenden Abfälle und der überschüssigen Nährstoffe, wobei zwei der genannten Paare an die Verteilungsrillen aufweisenden Endwänden angeschlossen sind und das dritte zwischen den beiden planen Filtermembranen angeschlossen ist;
b) Zirkulationseinrichtungen des ersten dynamischen flüssigen Mediums F1 als Kreislauf in Form einer geschlossenen Schleife und ein das genannte Medium enthaltendes Expansionsgefäß R1, wobei diese Einrichtungen mit dem genannten Kulturraum verbunden sind;
c) Zirkulationseinrichtungen des zweiten dynamischen flüssigen Mediums F2 als Kreislauf in geschlossener Schleife oder in offener Schleife, abhängig von der jeweiligen Funktion des genannten Mediums, wobei diese Einrichtungen mit dem genannten Kulturraum verbunden sind;
d) Zirkulationseinrichtungen des dritten dynamischen flüssigen Mediums F3 gemäß einem Kreislauf in offener Schleife und einem das genannte Medium enthaltenden Speicher R3, wobei diese Einrichtungen mit dem genannten Kulturraum verbunden sind;
e) Einrichtungen zur Kontrolle und Regelung sowie solche zur Konditionierung der dynamischen flüssigen Medien, verbunden mit einer Regelungs-Steuerungsgruppe.

21. Bioreaktor nach Anspruch 20, **dadurch gekennzeichnet, dass** die Zirkulationseinrichtungen der genannten flüssigen Medien F1, F2 und F3 jeweils mit einer Pumpe P1, P2 und P3 ausgerüstet sind, die mit der Regelungs-Steuerungsgruppe verbunden sind.

22. Bioreaktor nach den Ansprüchen 20 und 21, **dadurch gekennzeichnet, dass** die Zirkulationseinrichtungen des dritten dynamischen flüssigen Mediums F3 mit einem mit der Regelungs-Steuerungsgruppe verbundenen Ventil V3 ausgerüstet sind.

23. Bioreaktor nach Anspruch 20, **dadurch gekennzeichnet, dass** das Expansionsgefäß R1 das wachstumsfaktorenreiche dynamische flüssige Medium F1 enthält und mit einem Filter mit einer 0,22µm-Durchlassschwelle und einem Eletroventil V1 ausgestattet ist, das durch die Regelungs-Steuerungsgruppe gesteuert wird.

24. Bioreaktor nach wenigstens einem der Ansprüche 20 bis 23, **dadurch gekennzeichnet, dass** das Expansionsgefäß R1 mit Sensoren für ein oberes Niveau und ein unteres Niveau ausgestattet ist, die dazu dienen, die Umkehrung der Zirkulation der Fluide im Innern des genannten Kulturraums auszulösen.

25. Bioreaktor nach Anspruch 20, **dadurch gekennzeichnet, dass** die Konditionierungseinrichtungen der dynamischen flüssigen Medien Einrichtungen zur Regelung des Sauerstoffgehalts, der Temperatur und des pH-Werts dieser Medien umfassen.

26. Bioreaktor nach Anspruch 25, **dadurch gekennzeichnet, dass** die Konditionierungseinrichtungen der dynamischen flüssigen Medien funktionelle Einheitsmodule sind, dimensioniert für einen bestimmten Massen- oder Wärmeenergietransferwert.

27. Bioreaktor nach Anspruch 26, **dadurch gekennzeichnet, dass** die funktionellen Module Belüfter, Wärmeaustauscher, pH-Regulatoren oder auch Module zur Dialyse, Ultrafiltrierung sind.

28. Bioreaktor nach den Ansprüchen 20 bis 27, **dadurch gekennzeichnet, dass** er einen Kulturraum nach wenigstens einem der Ansprüche 1 bis 19 umfasst.

29. Bioreaktor nach wenigstens einem der Ansprüche 20 bis 28, **dadurch gekennzeichnet, dass** ein Druck p1 in der Zone C1 des genannten Kulturraums herrscht, die sich zwischen der oberen Endwand des genannten Raums und der planen Filtermembran mit der Durchlassschwelle 0,12 µm bis 0,44 µm befindet.

30. Bioreaktor nach wenigstens einem der Ansprüche 20 bis 29, **dadurch gekennzeichnet, dass** ein Druck p2 in der Zone C2 des Kulturraums herrscht, die sich zwischen den beiden planen Filtermembranen mit unterschiedlichen Durchlassschwellen befindet.

31. Bioreaktor nach wenigstens einem der Ansprüche 20 bis 30, **dadurch gekennzeichnet, dass** ein Druck p3 in der Zone C3 des genannten Kulturraums herrscht, die sich zwischen der planen Filtermembran mit der Durchlassschwelle von höchstens 15 kDa und der unteren Endwand der Umhüllung des genannten Raums befindet.

32. Bioreaktor nach wenigstens einem der Ansprüche 20 bis 31, **dadurch gekennzeichnet, dass** das wachstumsfaktorenreiche Nährmedium F1 aus der Zone C1 des Kulturraums in die Zone C3 des genannten Kulturraums dräniert wird, wenn der Druck p1 größer ist als der Druck p3, und dass der Druck p2 in dem Kulturraum zwischen p1 und p3 enthalten ist.

33. Bioreaktor nach wenigstens einem der Ansprüche 20 bis 31, **dadurch gekennzeichnet, dass** das wachstumsfaktorenlose Nährmedium F3 aus der Zone C3 des Kulturraums in die Zone C1 des genannten Kulturraums dräniert wird, wenn der Druck p3 größer ist als der Druck p1, und dass der Druck p2 in dem Kulturraum zwischen p1 und p3 enthalten ist.

34. Bioreaktor nach wenigstens einem der Ansprüche 20 bis 32, **dadurch gekennzeichnet, dass** die Öffnung bzw. Inbetriebnahme der Pumpe P1 die Versorgung des Kulturraums mit wachstumsfaktorenreichem Nährmedium F1 ermöglicht, wobei die Pumpe P3 stillsteht, und dass das Gegendruckventil V3 so geregelt bzw. eingestellt ist, dass der in der Zone C1 des genannten Kulturraums herrschende Druck p1 höher ist als der in der Zone C3 des genannten Raums herrschende Druck p3, wissend dass der in der Zone C2 herrschende Druck p2 zwischen den Drücken p1 und p3 enthalten ist.

35. Bioreaktor nach Anspruch 34, **dadurch gekennzeichnet, dass** der gemäß einer speziellen Sequenz programmierte Regelungs-Steuerungsblock die Zirkulationsrichtung der Flüsse umkehrt, indem er die Pumpe P1 ausschaltet und die Pumpe P3 einschaltet, sobald das in dem Expansionsgefäß R1 enthaltene Volumen ein unteres Niveau erreicht.

36. Bioreaktor nach Anspruch 35, **dadurch gekennzeichnet, dass** das Ventil V3 geschlossen ist.

37. Bioreaktor nach den Ansprüchen 35 und 36, **dadurch gekennzeichnet, dass** das wachstumsfaktorenlose Grundstoff-Nährmedium die Neuversorgung des Kulturraums ermöglicht, wo bzw. wenn der Druck p3 in der Zone C3 des genannten Raums höher ist als der in der Zone C1 herrschende Druck p1, wissend dass der in der Zone C2 herrschende Druck p2 zwischen den Drücken p1 und p3 enthalten ist.

38. Bioreaktor nach wenigstens einem der Ansprüche 20 bis 37, **dadurch gekennzeichnet, dass** die Pumpen P1 und P3 stillstehen, wenn die Eingangs- und Ausgangszirkulationskonfiguration des zweiten dynamischen flüssigen Mediums F2 in Abhängigkeit von der ihr zugeteilten Funktion als geschlossener oder offener Kreislauf festgelegt ist.

39. Bioreaktor nach Anspruch 38, **dadurch gekennzeichnet, dass** das dynamische flüssige Medium F2 zur Einführung der zu kultivierenden Zellen und der Rückgewinnung der kultivierten Zellen dient.

40. Bioreaktor nach Anspruch 38, **dadurch gekennzeichnet, dass** das dynamische flüssige Medium F2 zur Einführung von Genen in den die Zellkultur enthaltenden Kulturraum und bei der Rückgewinnung der kultivierten, genetisch modifizierten Zellen als Transfervektor dient.

41. Bioreaktor nach Anspruch 38, **dadurch gekennzeichnet, dass** das dynamische flüssige Medium F2 zur Einführung einer Flüssigkeit zum Ausspülen der die Entwicklung der Zellkultur hemmenden Moleküle und zur Elimination dieser Moleküle dient.

42. Bioreaktor nach Anspruch 39, **dadurch gekennzeichnet, dass** für die Inokulation des genannten Mediums F2 in der Zone C2 des Zellkulturraums durch ein biokompatibles Septum hindurch, mit Hilfe einer Spritze, das Elektroventil V2E1 geöffnet wird, während die Elektroventile V2E2, V2E3, V2S1, V2S2 und V2S3 geschlossen werden.

43. Bioreaktor nach Anspruch 39, **dadurch gekennzeichnet, dass** für die Rückgewinnung der Zellen nach der Kultur aus dem Kulturraum das Elektroventil V2S1 geöffnet wird, die Elektroventile V2E2, V2E3, V2S2 und V2S3 geschlossen werden und die Pumpe P2 in Betrieb genommen wird, um den Inhalt der Zone C2 in den Zellensammelbehälter zu entleeren.

44. Bioreaktor nach Anspruch 41, **dadurch gekennzeichnet, dass** - zur Elimination der entwicklungshemmenden Moleküle - wenn das zweite Medium F2 die zum Spülen des Zelleneinschließungsraums nötigen Elemente enthält, bei der Einführung dieses Mediums das Elektroventil V2E3 geöffnet ist und die Elektroventile V2E1, V2E2 geschlossen sind.

45. Bioreaktor nach Anspruch 41, **dadurch gekennzeichnet, dass** das zweite Spülmedium F2 kontinuierlich in einer offenen Schleife zirkuliert, und dass bei der Einführung und der Rückgewinnung dieses Spülmediums die Elektroventile V2E1, V2E2, V2S1, V2S2 geschlossen sind und die Elektroventile V2E3, V2S3 geöffnet sind.

46. Bioreaktor nach Anspruch 40, **dadurch gekennzeichnet, dass** für die Inokulation der genetischen Transfervektoren, enthalten in dem in einem geschlossenen Kreislauf in der Zone C2 des Kulturraums zirkulierenden Medium F2, das Elektroventil V2E2 geöffnet wird, während die Elektroventile V2E1, V2E3, V2S1, V2S3 geschlossen werden.

47. Bioreaktor nach wenigstens einem der Ansprüche 20 bis 46, **dadurch gekennzeichnet, dass** die Regelungs-Steuerungsgruppe die Gesamtheit der Informationen bezüglich der dynamischen flüssigen Medien F1, F2, F3 - geliefert durch die Kontroll- und Regelungseinrichtungen - und die Informationen bezüglich der diversen Speicher bzw. Behälter, Pumpen, Ventile und der in den Zonen C1, C2, C3 des Kulturraums herrschenden Drücke erhält, sie verarbeitet und die nötigen Betriebsbefehle aussendet.

## Claims

1. Culture chamber for the extracorporeal culture of animal cells, delimited by a casing with an axis of symmetry formed by an external lateral wall, two end walls and dynamic liquid media inlets and outlets, **characterised in that** it comprises :
a) at least two plane filtering membranes with a different cut-off threshold, perpendicular to the axis of symmetry ;
b) between the membranes, a bed of biocompatible macrosubstrates favouring adhesion of the cells under culture conditions ;
c) two end walls provided with dynamic liquid medium distribution grooves ;
d) three dynamic liquid medium inlet and outlet pairs, intended to supply the cells culture chamber and selectively extract the cultured cells, the waste resulting from the culture thereof and excess nutrients, wherein two of the pairs are connected to the end walls provided with distribution grooves, the third being connected between the two plane filtering membranes.

2. Culture chamber according to claim 1, **characterised in that** one of the plane filtering membranes has a cut-off threshold between 0.12 µm and 0.44 µm, and the other plane filtering membrane has a cut-off threshold of not more than 15 KiloDalton (KDa).

3. Culture chamber according to one at least of claims 1 or 2, **characterised in that** one of the plane filtering membranes has a cut-off threshold between 0.18 µm and 0.26 µm and the other plane filtering membrane has a cut-off threshold between 10 and 12 KDa.

4. Culture chamber according to one at least of claims 1 to 3, **characterised in that** the two membranes with different cut-off thresholds are at a distance of not more than 400 µm from each other.

5. Culture chamber according to one at least of claims 1 to 4, **characterised in that** the macrosubstrates present between the membranes have a cylindrical, polyhedral or spherical shape.

6. Culture chamber according to one at least of claims 1 to 5, **characterised in that** the constituent substance of the macrosubstrates is selected from the group of mineral substances, metallic substances and polymer substances.

7. Culture chamber according to claim 6, **characterised in that** the constituent substance of the macrosubstrates is selected from the group consisting of coral, titanium and alloys thereof, polyamides.

8. Culture chamber according to one at least of claims 1 to 7, **characterised in that** the bed formed of macrosubstrates placed between two membranes with a different cut-off threshold is not more than 400 µm thick.

9. Culture chamber according to one at least of claims 1 to 8, **characterised in that** the distribution grooves of the end walls are organised into a main network, said grooves being preferentially divergent in the direction of dynamic liquid medium propagation.

10. Culture chamber according to one at least of claims 1 to 9, **characterised in that** the distribution grooves of the end walls are completed by a secondary network formed by secondary grooves, which are appreciably perpendicular to the grooves of the main network.

11. Culture chamber according to one at least of claims 9 or 10, **characterised in that** the main and secondary network form a gridded dynamic liquid medium propagation network.

12. Culture chamber according to one at least of claims 1 to 11, **characterised in that** the grooves of the main network and/or the secondary network of the end walls are in contact with the membranes with different cut-off levels.

13. Culture chamber according to one at least of claims 9 to 12, **characterised in that** the grooves of the main network are not more than 5 mm deep, not more than 2 mm wide, and the pitch between two adjacent grooves is not more than 2 mm.

14. Culture chamber according to one at least of claims 9 to 12, **characterised in that** the grooves of the secondary network are not more than 2 mm deep and not more than 2 mm wide.

15. Culture chamber according to one at least of claims 1 to 14, **characterised in that** one of the two dynamic liquid medium inlet and outlet pairs F1 is connected at an end wall and supplies the cell culture medium, via the distribution grooves, with a nutrient medium comprising growth factors through the plane filtering membrane with a cut-off threshold between 0.12 and 0.44 µm.

16. Culture chamber according to claim 15, **characterised in that** the inlet and outlet pair of the dynamic liquid medium F1 operates in a closed circuit according to a loop.

17. Culture chamber according to one at least of claims 1 to 16, **characterised in that** the inlet and outlet pair of the dynamic liquid medium F2, connected between the two filtering membranes, carries out the functions consisting of introducing the cells to be cultured and retrieving said cultured cells, transferring genes and retrieving the genetically modified cells, and introducing a flushing liquid and eliminating molecules inhibiting the growth of the cells in culture.

18. Culture chamber according to one at least of claims 1 to 17, **characterised in that** the other of the two inlet and outlet pairs of the dynamic liquid medium F3 is connected in an open circuit at the other end wall and supplies, via the distribution grooves, the cell culture medium with a growth factor-free nutrient medium, through the plane filtering membrane with a cut-off threshold of not more than 15 KDa.

19. Culture chamber according to one at least of claims 1 to 18, **characterised in that** the three dynamic liquid media inlet and outlet pairs are placed in the three vertical planes passing through the axis of symmetry of the casing, said planes being offset by an angle of approximately 60° between the first inlet and the second inlet, and an angle of approximately 120° between the first and third dynamic liquid media inlet, the outlets of said liquid media being in the same angular arrangements.

20. Bioreactor for the extracorporeal culture of animal cells comprising a culture chamber delimited by a casing with an axis of symmetry formed by an external lateral wall, two end walls and dynamic liquid media inlets and outlets and comprising circulation means of said media in said chamber, **characterised in that** it comprises:
a) a culture chamber for said cells comprising at least two plane filtering membranes with a different cut-off threshold, perpendicular to the axis of symmetry and between said membranes with a different cut-off threshold is located a bed of biocompatible macrosubstrates favouring adhesion of the cells under culture conditions, said chamber being delimited by a casing with an axis of symmetry comprising two end walls provided with dynamic liquid media distribution grooves and three inlet and outlet pairs of the dynamic liquid media F1, F2, F3, intended to supply the cell culture chamber and selectively extract the cultured cells, the waste resulting from the culture thereof and excess nutrients, wherein two of the pairs are connected to the end walls provided with distribution grooves, the third being connected between the two plane filtering membranes ;
b) circulation means of the first dynamic liquid medium F1 along a closed loop circuit and an expansion vessel R1 containing said medium, said means being connected to said culture chamber ;
c) circulation means of the second dynamic liquid medium F2 along a closed loop or open loop circuit according to the function attributed to said medium and a container R2 containing said medium, said means being connected to said culture chamber ;
c) circulation means of the third dynamic liquid medium F3 along an open loop circuit and a container R3 containing said medium, said means being connected to said culture chamber ;
e) dynamic liquid media monitoring and control and conditioning means, connected to a monitoring and control unit.

21. Bioreactor according to claim 20, **characterised in that** the circulation means of said liquid media F1, F2 and F3 are equipped with a pump P1, P2 and P3 respectively, connected to the monitoring and control unit.

22. Bioreactor according to claims 20 and 21, **characterised in that** the circulation means of the third dynamic liquid medium F3 are equipped with a valve V3 connected to the monitoring and control unit.

23. Bioreactor according to claim 20, **characterised in that** the expansion vessel R1 containing the growth factor-rich dynamic liquid medium F1 is equipped with a filter having a cut-off threshold of 0.22 µm and an electrovalve VI controlled by the monitoring and control unit.

24. Bioreactor according to one at least of claims 20 to 23, **characterised in that** the expansion vessel R1 is equipped with liquid medium high level and low level sensors, which are used to trigger fluid circulation inversion within said culture chamber.

25. Bioreactor according to claim 20, **characterised in that** the dynamic liquid media conditioning means comprise means to adjust the oxygen, temperature and pH of said media.

26. Bioreactor according to claim 25, **characterised in that** the conditioning means of said media are functional unit modules, designed for a specific mass or thermal energy transfer value.

27. Bioreactor according to claim 26, **characterised in that** the functional modules are aerators, heat exchangers, pH regulators, or dialysis, ultrafiltration modules.

28. Bioreactor according to claims 20 to 27, **characterised in that** it comprises a culture chamber according to one at least of claims 1 to 19.

29. Bioreactor according to one at least of claims 20 to 28, **characterised in that** a pressure p1 is applied in the zone C1 of the culture chamber, located between the upper end wall and the plane filtering membrane with a cut-off threshold between 0.12 µm and 0.44 µm, of said chamber.

30. Bioreactor according to one at least of claims 20 to 29, **characterised in that** a pressure p2 is applied in the zone C2 of the culture chamber comprised between the two plane filtering membranes with different cut-off thresholds of said chamber.

31. Bioreactor according to one at least of claims 20 to 30, **characterised in that** a pressure p3 is applied in the zone C3 of the culture chamber, located between the plane filtering membrane with a cut-off threshold of not more than 15 KDa and the lower end wall of the casing of said chamber.

32. Bioreactor according to one at least of claims 20 to 31, **characterised in that** the growth factor-rich nutrient medium F1 is drained from the zone C1 of the culture chamber to the zone C3 of said culture chamber when the pressure p1 is greater than the pressure p3 and that the pressure p2 is between p1 and p3 in the culture chamber.

33. Bioreactor according to one at least of claims 20 to 31, **characterised in that** the growth factor-free nutrient medium F3 is drained from the zone C3 of the culture chamber to the zone C1 of said culture chamber when the pressure p3 is greater than the pressure p1 and that the pressure p2 is between p1 and p3 in the culture chamber.

34. Bioreactor according to one at least of claims 20 to 32, **characterised in that** the opening of the pump P1 enables the supply of the culture chamber with growth factor-rich nutrient medium F1, the pump P3 being shut down, and that the back-pressure valve V3 is set such that the pressure p1 applied in the zone C1 of said culture chamber is greater than the pressure p3 applied in the zone C3 of said chamber, given that the pressure p2 applied in the zone C2 is between the pressures p1 and p3.

35. Bioreactor according to claim 34, **characterised in that** the monitoring and control unit programmed according to a specific sequence inverts the direction of circulation of the flows by shutting down the pump P1 and starting up the pump P3 when the volume contained in the expansion vessel R1 reaches a low level.

36. Bioreactor according to claim 35, **characterised in that** the valve V3 is closed.

37. Bioreactor according to claims 35 and 36 **characterised in that** the growth factor-free basic nutrient medium F3 enables the replenishment of the culture chamber where the pressure p3 in the zone C3 of said chamber is greater than the pressure p1 applied in the zone C1, given that the pressure p2 applied in the zone C2 is between the pressures p1 and p3.

38. Bioreactor according to one at least of claims 20 to 37, **characterised in that** the pumps P1 and P3 are shut down when the circulation inlet and outlet configuration of the second dynamic liquid medium F2 is set to a closed or open circuit according to the function attributed.

39. Bioreactor according to claim 38, **characterised in that** the dynamic liquid medium F2 is used to introduce the cells to be cultured into the culture chamber and recover the cells after culture.

40. Bioreactor according to claim 38, **characterised in that** the dynamic liquid medium F2 is used to introduce the gene transfer vector into the culture chamber containing the cells in culture and recover the genetically modified cultured cells.

41. Bioreactor according to claim 38, **characterised in that** the dynamic liquid medium F2 is used to introduce a flushing liquid for the molecules inhibiting the growth of the cells in culture and eliminate said molecules.

42. Bioreactor according to claim 39, **characterised in that** for the inoculation of said medium F2 in the zone C2 of the cell culture chamber via a biocompatible septum using a syringe, the electrovalve V2E1 is open, whereas the electrovalves V2E2, V2E3, V2S1, V2S2 and V2S3 are closed.

43. Bioreactor according to claim 39, **characterised in that** for the retrieval of the cells after culture in the culture chamber, the electrovalve V2S1 is opened, the electrovalves V2E2, V2E3, V2S2, and V2S3 are closed and the pump P2 is started up to drain the contents of the zone C2 in the cell collection pouch.

44. Bioreactor according to claim 41, **characterised in that** for the elimination of the inhibitory molecules when the second medium F2 contains the elements required for rinsing the cell confinement volume, during the introduction of said medium, the electrovalve V2E3 is opened and the electrovalves V2E1, V2E2 are closed.

45. Bioreactor according to claim 41, **characterised in that** said second rinsing medium F2 circulates continuously in an open loop and that during the introduction and retrieval of said rinsing medium, the electrovalves V2E1, V2E2, V2S1, V2S2 are closed, the electrovalves V2E3, V2S3 are open.

46. Bioreactor according to claim 40, **characterised in that**, for the inoculation of the gene transfer vectors contained in said medium F2 circulating in a closed circuit in the zone C2 of the cell culture chamber, the electrovalve V2E2 is opened, whereas the electrovalves V2E1, V2E3, V2S1, V2S3 are closed.

47. Bioreactor according to one at least of claims 20 to 46, **characterised in that** the monitoring and control unit receives all the information relating to the dynamic liquid media F1, F2, F3, by the monitoring and control means, and the information relating to the various containers, pumps, valves and pressures applied in the zones C1, C2, C3 of the culture chamber, handles them and transmits the operating orders required.
